(19) Europäisches Patentamt
European Patent Office
Office européen des brevets

(11) **EP 1 312 304 B1**

(12) **EUROPEAN PATENT SPECIFICATION**

(45) Date of publication and mention
of the grant of the patent:
**03.09.2008 Bulletin 2008/36**

(51) Int Cl.:
***A61B 5/04*** (2006.01)

(21) Application number: **02022202.2**

(22) Date of filing: **01.10.2002**

(54) **Biomagnetic field measuring apparatus**

Messgerät für ein biomagnetisches Feld

Appareil de mesure d'un champ biomagnétique

(84) Designated Contracting States:
**DE FR GB**

(30) Priority: **16.11.2001 JP 2001350920**

(43) Date of publication of application:
**21.05.2003 Bulletin 2003/21**

(73) Proprietors:
• **Hitachi, Ltd.**
**Chiyoda-ku,**
**Tokyo 100-8010 (JP)**
• **Hitachi High-Technologies Corporation**
**Tokyo 105-0003 (JP)**

(72) Inventors:
• **Suzuki, Hiroyuki**
**Hitachinaka-shi,**
**Ibaraki 312-0062 (JP)**
• **Tobita, Tomoyuki**
**Hitachinaka-shi,**
**Ibaraki 312-0063 (JP)**

• **Miyashita, Tsuyoshi**
**Fuchuu-shi,**
**Tokyo 183-0042 (JP)**
• **Tsukada, Keiji**
**Kashiwa-shi,**
**Chiba 277-0084 (JP)**

(74) Representative: **Strehl Schübel-Hopf & Partner**
**Maximilianstrasse 54**
**80538 München (DE)**

(56) References cited:
**WO-A-99/55228**      **US-B1- 6 195 576**

• **SALMINEN K ET AL: "CLASSIFICATION OF MAGNETOCARDIOGRAPHIC- AND COMBINED ELECTRO- MAGNETOCARDIOGRAPHIC MEASUREMENTS" PROCEEDINGS OF THE ANNUAL INTERNATIONAL CONFERENCE OF THE ENGINEERING IN MEDICINE AND BIOLOGY SOCIETY. PARIS, OCT. 29 - NOV. 1, 1992, NEW YORK, IEEE, US, vol. 2 CONF. 14, 29 October 1992 (1992-10-29), pages 577-578, XP000480544**

**Description**

BACKGROUND OF THE INVENTION

1. Field of the Invention

This invention relates to a biomagnetic field measuring apparatus for measuring the magnetic field of a living body that is generated by an electric current flowing in the living body, such as neural activity of the brain or myocardial activity of the heart of a living body. In particular, the invention relates to a biomagnetic field measuring apparatus equipped with a diagnosis support function with which a doctor diagnoses the brain disease and cardiac disease.

2. Description of the Related Art

A multi-channel biomagnetic imaging apparatus has been developed by using a superconducting quantum interference device (SQUID) which is a magnetic sensor in order to measure the distribution of very weak magnetic fields generated by the living body, to estimate the positions of active currents in the living body from the measured result and to image the distribution. Technologies related to the biomagnetic imaging apparatus have been disclosed in, for example, Japanese Patents Nos. 3140731 and 3140732.

The above related arts are concerned with the methods of efficiently and explicitly displaying the principle of operation of the device for displaying biomagnetic field diagram and the measured data, and are referring to the display of waveforms, display of magnetic field-strength diagram and display of magnetic field time-integration diagram. Diagnosing the cardiac disease relying only upon these displays, however, requires a high degree of knowledge related to the cardiac function and cardiac disease as well as knowledge concerning the principle of operation of cardiomagnetic measurement and a method of reconstituting the data. Therefore, medical doctors, in general, find it difficult to use the apparatus. Further, since a tremendous amount of data are obtained by the measurement, the display conditions for the diagnosis must be selected and set requiring extended periods of time.

Document US-B1-6195576 discloses a method and a system for producing magnetoencephalograms (MEG) in order to detect and to analyze magnetic effects of brain waves. An application of Mahalanobis distances to conventional electrocardiograms (ECG) is disclosed in WO-A-9955228.

SUMMARY OF THE INVENTION

It is an object of this invention to provide a biomagnetic field measuring apparatus equipped with a diagnosis support function capable of supporting the diagnosis by a doctor, preventing the looking-over of the diseased part and greatly shortening the diagnosing time by inferring or a to-be-tested person who is considered to be suffering from a cardiac disease by quantitatively grasping the features in the data measured by using the biomagnetic field measuring apparatus, and infering the candidate of disease based on the data measured from the to-be-tested person.

The present invention is defined in claim 1. Further advantageous features are set out in the dependent claims.

In order to achieve the above object, this invention is constituted as described below.

(1) A biomagnetic field measuring apparatus for measuring a magnetic field generated from the living body of a to-be-tested person at plural positions, wherein signal data measured by magnetic sensors are stored, a plurality of feature parameters are calculated from the stored signal data, and the calculated feature parameters are compared with a reference database formed based chiefly upon the results of measurement of the magnetic fields of healthy persons in order to discriminate whether the to-be-tested person is healthy.
As the reference database, there can be employed a variety of ones, such as a Mahalanobis space built up based on the data of healthy persons, a network representing a inference logic of neural network, etc.
(2) A biomagnetic field measuring apparatus for measuring a magnetic field generated from the living body of a to-be-tested person at plural positions, wherein signal data measured by magnetic sensors are stored, plural feature parameters are calculated from the stored signal data, and the calculated feature parameters are compared and collated with a predetermined rule in order to discriminate whether the to-be-tested person is healthy.
The predetermined rule is an expert system or the like formed by rule base in an if-then-else manner.
(3) A biomagnetic field measuring apparatus of (1) or (2) above, wherein the magnetic field to be measured is the one generated chiefly from the heart, and a feature parameter to be picked up is a direction of current near an R-wave peak at regular intervals.
(4) A biomagnetic field measuring apparatus according to (1) or (2) above, wherein the magnetic field to be measured is the one generated chiefly from the heart, and a feature parameter to be picked up is the one picked up from a so-called contor map of magnetic field-strength at a representative moment which is the one at which the amplitude becomes the greatest in the magnetic field intensity that is measured and in which points of an equal magnetic field are linked at the representative moment measured by the plural sensors.

(5) A biomagnetic field measuring apparatus for measuring a magnetic field generated from the living body of a to-be-tested person at plural positions, wherein the measured signal data are stored, plural feature parameters are calculated from the signal data that have been stored, a Mahalanobis space that will be described later in this specification is built up based upon the feature or characteristic parameters that are calculated, a Mahalanobis distance of the to-be-tested person in the Mahalanobis space is calculated based on plural feature parameters, and discrimination data calculation means calculates the discrimination data in order to discriminate whether the to-be-tested person is healthy relying upon the data of Mahalonobis distance.

(6) A biomagnetic field measuring apparatus according to any one of (1), (2) or (5), further comprising discrimination means which compares the discrimination data calculated by said discrimination data calculation means with the reference discrimination data that have been stored in advance for discriminating weather the to-be-tested person is healthy, and discriminates whether the to-be-tested person is healthy.

(7) A biomagnetic field measuring apparatus according to (6), wherein the reference discrimination data are arbitrarily set by the operator.

(8) A biomagnetic field measuring apparatus according to (6), further comprising a disease estimation function for displaying a candidate of disease based upon the plurality of feature parameters that are measured when it is discriminated by the discrimination means that the to-be-tested person is not healthy.

(9) A biomagnetic field measuring apparatus according to any one of (5) to (8), further comprising storage means for storing the feature parameters and Mahalanobis distances for each of the plurality of to-be-tested persons, and a function for displaying at least one feature parameter and Mahalanobis distance of a to-be-tested person who is selected.

(10) A biomagnetic field measuring apparatus according to any one of (5) to (8), further comprising storage means for storing the feature parameters and Mahalanobis distances for each of the plurality of to-be-tested persons, and a function for simultaneously displaying at least one feature parameter and Mahalanobis distance of a to-be-tested person who is selected, and a Mahalanobis distance and a feature parameter in the Mahalanobis space that is built up.

(11) A biomagnetic field measuring apparatus according to (9) or (10), further comprising a function for displaying an average value and a standard deviation in the Mahalanobis space of a to-be-tested person who is selected.

(12) A biomagnetic field measuring apparatus according to (9) or (10), further comprising a function for describing feature parameters of the to-be-selected person who is selected and the normalized characteristic feature parameter.

(13) A biomagnetic field measuring apparatus according to (9) or (10), further comprising a function for displaying, in an emphasized manner, the data of the to-be-tested person who has data of which the Mahalanobis distance is greater than that of the reference discrimination data that have been stored in advance in order to discriminate whether the to-be-tested person is healthy.

(14) A biomagnetic field measuring apparatus according to (9) or (10), further comprising a function for displaying, in an emphasized manner, the feature parameter of which the normalized feature parameter is greater than a predetermined value when the Mahalanobis distance is greater than that of the reference discrimination data that have been stored in advance for discriminating whether the to-be-tested person is healthy.

(15) A biomagnetic field measuring apparatus according to (9) or (10), further comprising a function for rearranging the data of plural to-be-tested persons that are stored in order of increasing Mahalanobis distances or decreasing Mahalanobis distances.

(16) A biomagnetic field measuring apparatus according to (5), further comprising storage means for storing plural Mahalanobis space data, and a function for selecting the space data that is used depending upon the data of the to-be-tested person.

(17) A biomagnetic field measuring apparatus according to (16), further comprising a function for defining feature parameters for each of the space data.

(18) A biomagnetic field measuring apparatus according to any one of (1) to (5), wherein the magnetic field generated from the living body of the to-be-tested person is a cardiomagnetic field generated chiefly from the heart, and wherein there is further provided a function for displaying, on a screen, a contour map of magnetic field-strength diagram close to P-waves, QRS-waves and T-waves among the cardiomagnetic field signal data.

BRIEF DESCRIPTION OF THE DRAWINGS

Fig. 1 is a view schematically illustrating the constitution of an embodiment of a biomagnetic field measuring apparatus which the invention puts into practice;
Fig. 2 is a perspective view illustrating the constitution of a magnetic sensor arrangement used for the biomagnetic field measuring apparatus of Fig. 1;

Fig. 3 is a perspective view of a single magnetic sensor for detecting the normal component in the magnetic field and of a single magnetic sensor for detecting the tangential component in the magnetic field, that are used for the biomagnetic field measuring apparatus of Fig. 1;

Fig. 4 is a diagram illustrating a positional relationship between the magnetic sensors in the biomagnetic field measuring apparatus of Fig. 1 and the chest of a to-be-tested person;

Fig. 5 is a diagram showing time-waveforms of tangential components in the cardiomagnetism of a particular channel measured from a healthy person by using a biomagnetic field measuring apparatus of Fig. 1;

Fig. 6 is a diagram showing magnetic field-strength diagrams near the peaks of P-wave, R-wave and T-wave formed from the cardiomagnetic data measured from a healthy person by using the biomagnetic field measuring apparatus of Fig. 1;

Fig. 7 is a magnetic field-strength diagram in the QRS-waves measured from a patient suffering from myocardial infarction by using the biomagnetic field measuring apparatus of Fig. 1;

Fig. 8 is a magnetic field-strength diagram in the QRS-waves measured for the right leg block by using the biomagnetic field measuring apparatus of Fig. 1;

Fig. 9 is a table showing feature parameters for quantitatively evaluating the cardiomagnetic signals;

Fig. 10 is a diagram of distribution of Mahalanobis distances obtained concerning a healthy person and a patient of cardiac disease;

Fig. 11 is a functional block diagram of a program system executed by a computer 8-1 in the biomagnetic field measuring apparatus of Fig. 1;

Fig. 12 is a diagram illustrating a basic layout of a display screen displayed on a display unit of the biomagnetic field measuring apparatus of Fig. 1;

Fig. 13 is a diagram illustrating the operation menu in the menu bar portion on the display screen displayed on the display unit in a privileged user mode in the biomagnetic field measuring apparatus of Fig. 1;

Fig. 14 is a diagram of layout of a data display region in the basic layout on the display screen displayed on the display unit of the biomagnetic field measuring apparatus of Fig. 1;

Fig. 15 is a flowchart illustrating the whole operation conducted in the biomagnetic field measuring apparatus of Fig. 1;

Fig. 16 is a flowchart illustrating the measurement of data at step for measuring the data in the operation flowchart of Fig. 13;

Fig. 17 is a flowchart illustrating the analysis of data at step for analyzing the data in the operation flowchart of Fig. 14;

Fig. 18 is a view showing the screen of a list of to-be-testedpersons shown on the display unit of the biomagnetic field measuring apparatus of Fig. 1;

Fig. 19 is a diagram illustrating the content of a dialog box for registering the to-be-tested person, which is opened as an operation menu on the display screen on the display unit when the "List of To-Be-Tested Persons (L)" - "Register the To-Be-Tested Person (R)" are selected in the biomagnetic field measuring apparatus of Fig. 1.;

Fig. 20 is a diagram illustrating the content of a dialog box for registering the to-be-tested person, which is opened as an operation menu on the display screen on the display unit when the "List of To-Be-Tested Persons (L)" - "Register the To-Be-Tested Person (R) " are selected in the biomagnetic field measuring apparatus of Fig. 1;

Fig. 21 is a diagram of a screen for measuring the data displayed on the display unit of the biomagnetic field measuring apparatus of Fig. 1;

Fig. 22 is a diagram of a screen for displaying magnetic field-strength diagrams on the display unit of the biomagnetic field measuring apparatus of Fig. 1;

Fig. 23 is a diagram of a screen for displaying time-integral diagrams displayed on the display unit of the biomagnetic field measuring apparatus of Fig. 1;

Fig. 24 is a diagram of a screen for displaying the summary on the display unit of the biomagnetic field measuring apparatus of Fig. 1;

Fig. 25 is a factor effect diagram;

Fig. 26 is a diagram of effective parameters for each of the patients;

Fig. 27 is a block diagram of when a neural network is used;

Fig. 28 is a diagram showing a model of a neuron (nerve cell) which is a unit for processing the data; and

Fig. 29 is a diagram schematically illustrating a neural network of a hierarchical structure.

## DESCRIPTION OF THE PREFERRED EMBODIMENTS

Embodiments of the invention will now be described with reference to the drawings.

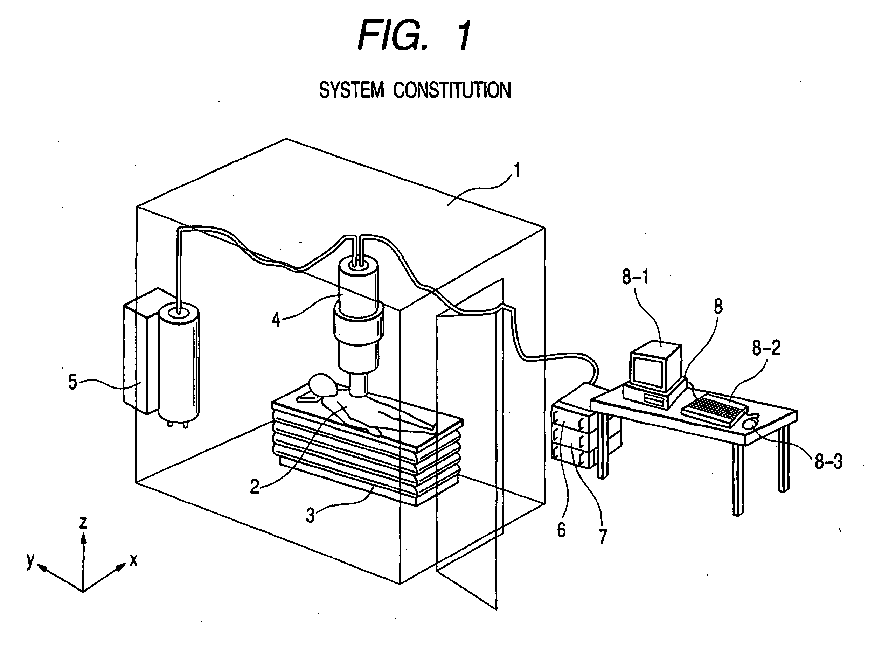

Fig. 1 is a view schematically illustrating the constitution of a biomagnetic field measuring apparatus according to an embodiment of the invention. In order to remove the effect of environmental magnetic noise, the biomagnetic field measuring apparatus is installed in a magnetical shielded room 1. A to-be-tested person 2 who is a living body is measured while lying on a bed 3 facing upward. The surface of the living body of the to-be-tested person (in the case of the chest, in general, the surface in parallel with the wall of chest) is nearly in parallel with the surface of the bed 3. This surface is in parallel with the plane x-y of the Cartesian coordinates system (x, y, z). The chest of the to-be-tested person is curved and is inclined but is, here, regarded to be nearly in parallel for simple explanation.

Over the chest of the to-be-tested person 2, there is disposed a dewar 4 filled with liquid helium which is a coolant. The dewar contains a plurality of magnetic sensors that include superconducting quantum interference devices (SQUIDs) and detection coils connected to the SQUIDs. Liquid helium is continuously replenished from the automatic replenishing device 5 placed outside the magnetical shielded room 1.

The output of the magnetic sensor is a voltage having a particular relationship to the intensity of the biomagnetic field (can be regarded to be a magnetic flux density) generated from the to-be-tested person 2 and is detected by the detection coil, and is input to an FLL (flux locked loop) circuit 6. In order to maintain constant the output of the SQUID, the FLL circuit 6 cancels the change in the biomagnetic field (biomagnetism) input to the SQUID through a feedback coil (this is called magnetic field lock). By converting the current flowing through the feedback coil into a voltage, it is allowed to obtain a voltage output having a particular relationship to a change in the biomagnetic field signals. Upon detecting the biomagnetic field through the feedback coil, even a very weak magnetic field can be detected maintaining a high sensitivity.

The output voltage is input to an amplifier/filter/amplifier (AFA) 7, and its output is sampled, subjected to the A/D conversion, and is received by a computer 8.

The computer 8 is a personal computer, wherein 8-1 is a display unit, 8-2 is a keyboard, and 8-3 is a mouse. The mouse 8-3 is used for moving a cursor on the screen to select an object that is to be treated. This operation can also be done by operating the keyboard. The AFA 7 can be adjusted for its input gain (Igain) and output gain (Ogain). The AFA 7, further, includes a low-pass filter (LPF) for passing frequency signals of lower than a first reference frequency, a high-pass filter (HPF) for passing frequency signals of higher than a second reference frequency but is lower than the first reference freuency, and a notch filter or Band Eliminator Filter (BEF) for cutting the commercial power source frequency. The computer 8 is capable of executing a variety of processings, and the processed results are displayed on the display unit 8-1.

As the SQUID, for example, there is used a DC SQUID. A DC bias current (Ibias) is supplied to the SQUID so as to produce a voltage (V) corresponding thereto when an external magnetic field is given to the SQUID. When the external magnetic field is expressed by a magnetic flux $\Phi$, a characteristics curve of V for $\Phi$ is given by a periodic function, i.e., a $\Phi$-V characteristics curve is given by a periodic function. Prior to taking the measurement, the offset voltage (VOFF) of the FLL circuit 6 is adjusted to bring the DC voltage in the $\Phi$-V characteristics curve to the zero level. Further, the offset voltage (AOFF) of the AFA 7 is so adjusted that the output of the AFA 7 becomes zero when the input to the AFA 7 is zero.

When a large magnetic field is applied to the SQUID from the external side, the magnetic field is trapped by the SQUID to hamper its normal operation. That is, observation of the time-waveform of the measured signals indicates that the base line of the signals is changing on the steps and that the noise is becoming extremely great. Further, observation of the $\Phi$-V characteristics of the SQUID sensor indicates that the characteristics curve of during the normal operation appears to be a periodic curve having a constant phase with respect to the scanning of $\Phi$. If the magnetic flux is trapped, however, the phase changes, and the amplitude of the characteristics curve becomes smaller than that of during the normal operation, from which it is allowed to detect the occurrence of the magnetic flux trapping. In this case, the SQUID is heated to once render it to be normally conducting. Thereafter, the heating is discontinued to remove the trapped magnetic field. The operation of heating the SQUID in this case is called heat flushing.

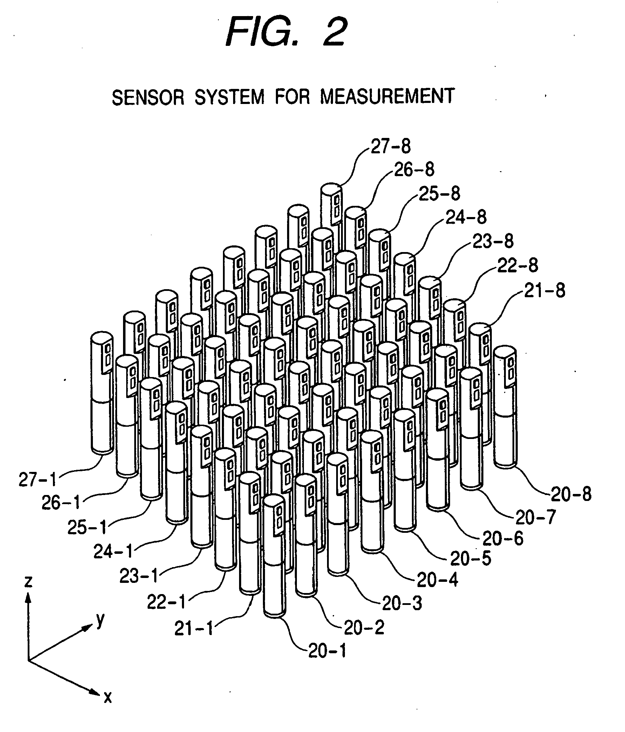

Fig. 2 illustrates the arrangement of magnetic sensors. The detection coils of the magnetic sensors include those coils for detecting the tangential component of the biomagnetic field (component nearly in parallel with the plane of the living body, i.e., nearly in parallel with the plane x-y) and those coils for detecting the normal component of the biomagnetic field (coil intersecting the plane of the living body, i.e., intersecting the plane x-y at right angles). As the coils for detecting the tangential component of the biomagnetic field, there are used two coils having coil planes facing the x-direction and the y-direction. As the coil for detecting the normal component in the biomagnetic field, there is used a coil having a coil plane facing the z-direction. As shown in Fig. 2, a plurality of magnetic sensors 20-1 to 20-8, 21-1 to 21-8, 22-1 to 22-8, 23-1 to 23-8, 24-1 to 24-8, 25-1 to 25-8, 26-1 to 26-8 and 27-1 to 27-8 are arranged like a matrix on a plane nearly in parallel with the plane of the living body, i.e., nearly in parallel with the plane x-y. The number of the magnetic sensors may be any. In Fig. 2, however, the matrix of the magnetic sensors consist of 8 rows and 8 columns; i.e., the number of the magnetic sensors is 8 x 8 = 64. As shown in Fig. 2, each magnetic sensor is so disposed that the lengthwise direction thereof is in agreement with a direction (z-direction) which is perpendicular to the plane of the living body, i.e., perpendicular to the plane x-y. In this embodiment, the bed surface is in parallel with the plane X-Y of the sensor. To improve the measuring precision, however, it is better to bring the magnetic sensors close to the body and incline them. However, the human body which is the to-be-tested person is moving at all times, and the detection units are caused to move if they are brought too close to the human body making it rather difficult to obtain the detection maintaining high precision.

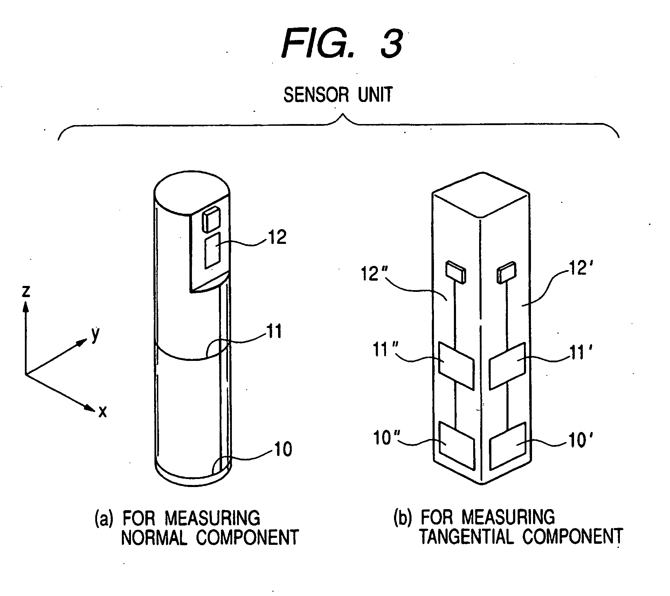

Fig. 3 illustrates the constitution of the sensor for detecting the normal component Bz in the biomagnetic field among the magnetic sensors, and wherein a coil formed of a superconducting wire (Ni-Ti wire) has a coil plane which is faced in the z-direction. The coil consists of a combination of two coils 10 and 11 facing in the directions opposite to each other, the coil 10 closer to the to-be-tested person 2 serving as a detection coil and the coil 11 on the remote side serving as a reference coil for detecting the external noise magnetic field. The external magnetic field noise is generated from a signal source farther than the to-be-tested person and, hence, the noise signal is detected by both the detection coil 10 and the reference coil 11. On the other hand, the magnetic field signal from the to-be-tested person is so weak that the biomagnetic field signal is detected by the detection coil 10 but the reference coil 11 does not almost respond to the biomagnetic field signal. Namely, the detection coil 10 detects the biomagnetic field signal and the external noise field signal, and the reference coil 11 detects the external noise field signal. Upon finding a difference in the signals detected by the two coils, therefore, it is allowed to measure the biomagnetic field maintaining a high S/N ratio. These coils are connected to the input coil of the SQUID through superconducting wires of amounting substrate mounting the SQUID 12 and, hence, the component Bz in the direction of normal of the detected biomagnetic field signal is transmitted to the SQUID.

The tangential components Bx, By of the biomagnetism can be detected by facing the detection coil plane and the reference coil plane in the x-direction or in the y-direction (not shown). Upon partially differentiating the normal component Bz obtained by the magnetic sensor of Fig. 3 concerning x and y, there can be obtained signals having a strong correlation to the real tangential components Bx, By. Therefore, a value obtained by partially differentiating the normal component Bz concerning x and y may be used as a tangential component. In this case, both the tangential components Bx, By and the normal component Bz can be detected and measured by one magnetic sensor.

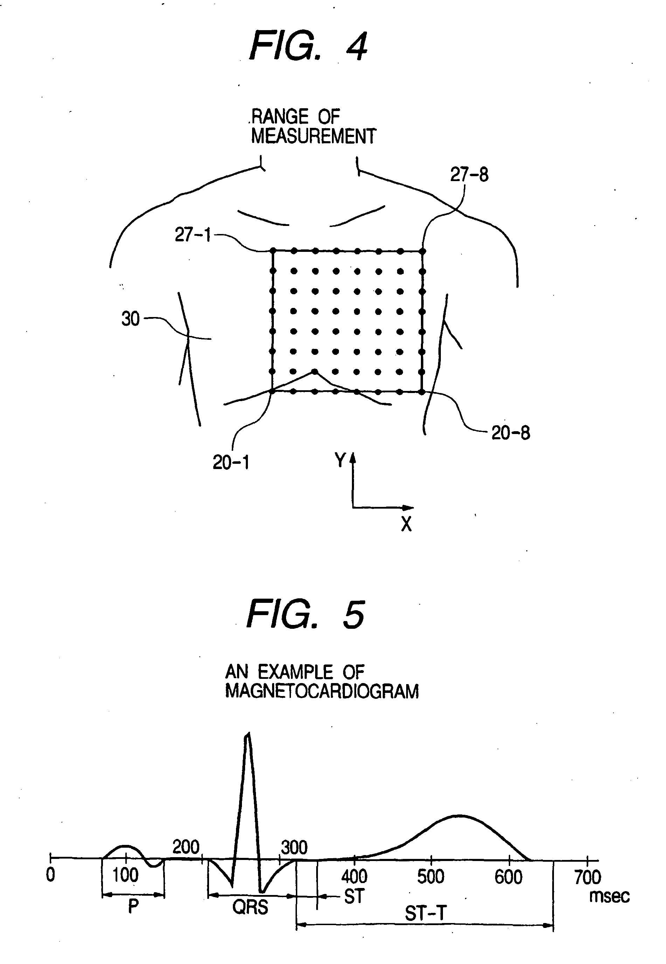

Fig. 4 illustrates a positional relationship between the magnetic sensors and the chest portion 30 which is a portion to be measured of the to-be-tested person 2. Dots represent points where the rows meet the columns on the matrix shown in Fig. 2, i.e., represent measuring points or measuring positions of the to-be-tested person 2. These measuring positions are also called channels. In this embodiment, as is understood from the figure, the direction of height of the to-be-tested person 2 is regarded to be the y-direction and the transverse direction of the to-be-tested person 2 is regarded to be the x-direction.

Fig. 5 shows a magnetocardiogram of a normal component Bz of a healthy person.

Like an electrocardiogram, the magnetocardiogram comprises P-waves due to an excited atrium, QRS-waves due to the step of excitation in the ventricle of the heart, and T-waves due to the step of recovery from the excitation in the venticle of the heat. It is considered that the signal due to excitation of the heart is nearly zero (0) between the P-wave and the Q-wave, and between the S-wave and the T-wave. The signal level among them is called base line. The time zones T1 in which the ventricle of the heat is depolarized, i.e., the times of peaks in the QRS waves in the contraction period, are represented as $t_Q$, $t_R$ and $t_S$. Further, the time zone of the T-waves during the step of re-polarization of the heat (period of expansion) is represented as T2.

Such time waveforms are simultaneously measured by 64 sensors. By drawing a magnetic field-strength diagram representing the signals (magnetic field intensities) measured by the sensors at a given moment in a two-dimensional manner, therefore, it is allowed to obtain a magnetic field distribution at that moment. In the drawing, the positions corresponding to the sensor positions are marked with arrows. This direction represents the direction of magnetic field found from the magnetic field data in a artificial-tangential direction obtained by partially differentiating, in the X- and Y-directions, the magnetic field data measured in the tangential direction or the magnetic field data in the normal direction. For easy explanation, here, the direction is turned by 90 degrees counterclockwise so as to be brought into agreement with the direction of the current for generating the magnetic field. The length of arrow represents the intensity of the magnetic field. Therefore, the arrow map in which the arrows are synthesized on the magnetic field-strength diagram in the tangential direction, represents the distribution of magnetic field depending upon the density of color and size of arrows, and the directions of currents are indicated by the directions of arrows.

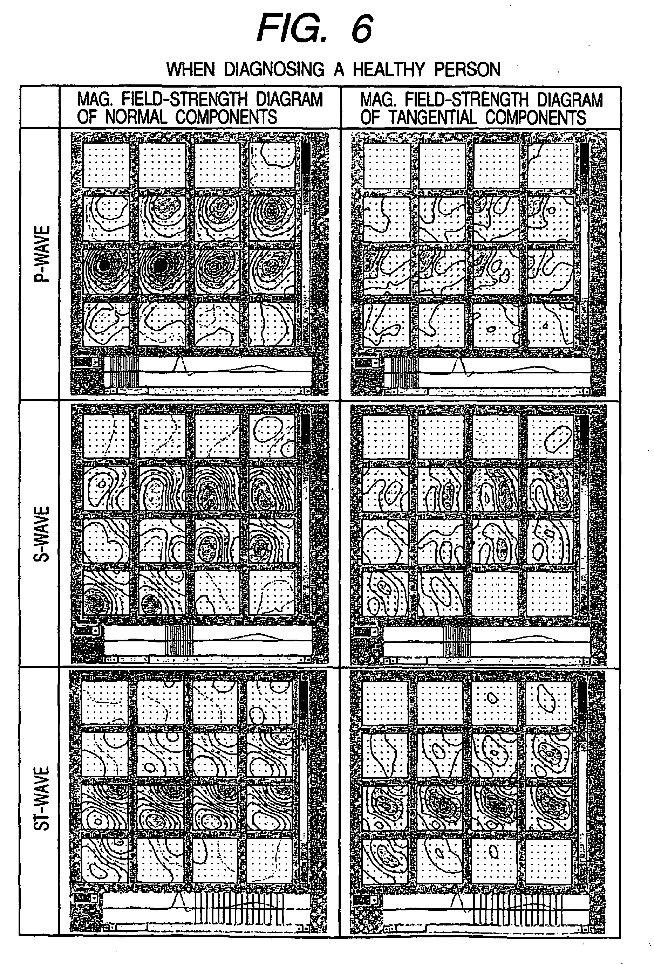

Fig. 6 is an arrow map based on the magnetic fields in the normal direction and in the tangential direction at every event of P-wave, peak (R) of QRS-wave and T-wave of a typical healthy person (37 years old, male). Figs. 6(a) and 6(b) illustrate the results of examining the changes in the magnetic field distribution at every event of P-wave, QRS-wave, and ST-T wave with the passage of time. These magnetic field-strength diagrams represent contour lines obtained by connecting the points of the same magnetic field intensities by lines. In the drawing, the arrows represent electric currents equivalent to the magnetic fields at the sensors.

From the normal components, it is learned that there are magnetic fields in two directions spewing out the magnetic field and sucking the magnetic field, and a current dipole can be imagined in the valley between the two poles. From the positions and directions, it is learned that the current dipole is moving downward from the right upper portion. This can be considered to be representing a phenomenon in which excitation is starting with the sinus of the right atrium. On the other hand, a view of the tangential component calculated from the normal component is expressing the place and direction of the portion that is estimated to have been excited in an easily discernible manner.

In the QRS-wave, the current dipole is initially estimated in the right lower direction. This direction soon changes into the left lower direction, and a large magnetic field is detected. After exhibited a maximum value in this direction, the magnetic field gradually attenuates. In the last period, the direction changes to the right upper direction and the magnetic field increases and, then, attenuates permitting QRS to terminate. If they are viewed as tangential components, the excited portion is at the center of the screen, and the current flows in the right lower direction. In the R-wave, the direction sharply changes, and the current starts flowing in the left lower direction. The current increases with the time and reaches the peak of the R-wave and, then, gradually attenuates. Then, as the S-wave is assumed, the current flows in the right upper direction and extinguishes. If these excitations are considered at the portions of the heart, it is estimated that the initial current of QRS is corresponding to the excitation which starts at the septum in the ventricle of the heart and moves to the right ventricle of the heart. In the R-wave, further, it is considered that the excitation shifts to the left ventricle, and the excitation appears very greatly from the magnitude of the cardiac muscle. The excitation that ends in the left ventricle then shifts toward the flow-out passage, and the step of depolarization in the ventricle ends.

In the ST-T wave, the ventricle is re-polarized, the excitation continues entirely toward the apex of the heart and, then, the intensity increases and, then, gradually attenuates. During this period, the direction does not almost change but the intensity only changes. This change can similarly be read from the normal components and the tangential components.

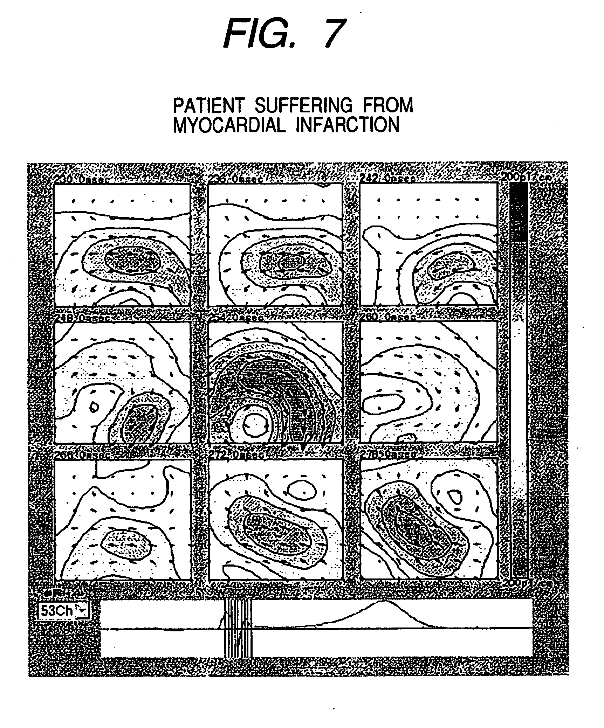

Fig. 7 is a magnetic field-strength diagram in the QRS wave of a male patient of 57 years old suffering from old myocardial infarction. The initial vector is directed leftward and is strong, and is considered to be corresponding to abnormal Q-wave in the electrocardiogram. Even in the T-wave, the directivity is different from that of a healthy person. In particular, the exciting portion is divided into two places, and the multi-dipole property is appearing conspicuously. Ischemiac cardiac disease can be diagnosed from the direction of current, change in the time and multi-dipole property on the magnetic field-strength diagram.

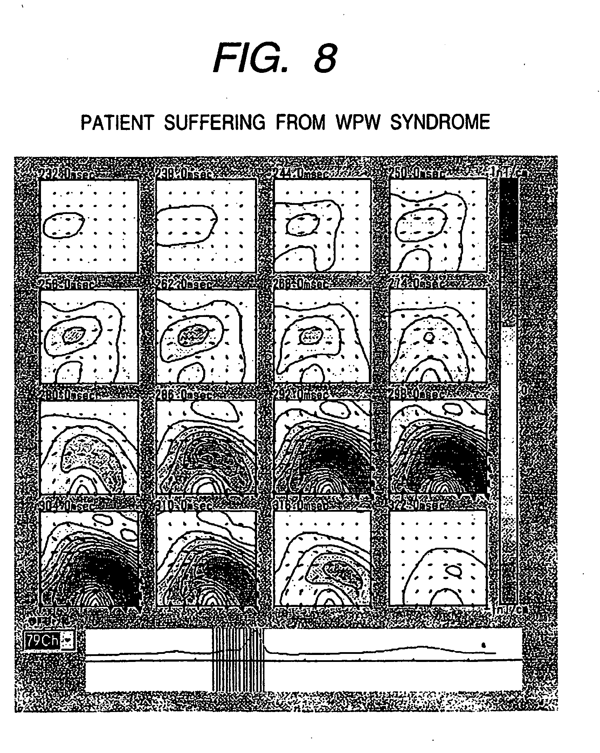

Fig. 8 is a magnetic field-strength diagram of a male patient of 22 years old suffering from WPW syndrome. The WPW syndrome is an abnormal condition in which there exists an abnormal sub-conduction passage in addition to the normal loculus conduction passage and the excitation from the atrium is quickly transmitted to the ventricle of the heart. When compared with the electrocardiogram of a healthy person, there is abnormal excitation due to the sub-conduction passage prior to the peak of R-wave, and the direction of current at the peak of R-wave is abnormal, too. Upon drawing the magnetic field-strength diagram, it is allowed to obtain electric physiological data in the heart, which cannot be learned from the time-waveforms.

According to this invention, feature parameters representing the features of the disease are automatically picked up from the magnetic field-strength diagram and the time-waveform by using a personal computer in order to discriminate whether the to-be-tested person is healthy or is suffering from some diseases relying upon the MTS system (Mahalanobis-Taguchi system). The MTS system has been closely disclosed in a literature "Quality Engineering for Developing Technology", Nippon Standard Association, but is briefly described below.

First, a to-be-tested person who has been known already to be a healthy person is measured to obtain a magnetocardiogram, and feature parameters $(Y_1, Y_2, ---, Y_n)$ are picked up from the data thereof. An average value m and a standard deviation $\sigma$ are found for all of the feature parameters, and the following conversion which is the standardization is effected for each of the items.

**[Mathematical 1]**

$$Y_i = (y_i - m)/\sigma$$

A Mahalanobis distance $D^2$ defined by the following formula is found concerning the standardized feature parameter $Y_i$.

**[Mathematical 2]**

$$D^2 = 1/k \times (\Sigma a_{ij} Y_i Y_j)$$

where $a_{ij}$ is a value of a component $a_{ij}$ in an inverse matrix of a correlation matrix, and $Y_1, Y_2, ---, Y_k$ are values of items of a number of k of an individual person who is normalized. In this case, the Mahalanobis distance $D^2$ becomes 1.0 in average.

The MTS system builds up a reference space based upon the multi-dimensional data obtained by adding

7

personal data such as sex, age, number of cigarettes smoked, number of years of smoking habit, amount of liquors drunk, number of years of drinking habit and degree of obesity to the results of urine testing and blood testing obtained in, for example, a group health examination. The MTS system, then, calculates the Mahalanobis distance for each of the to-be-tested persons. Here, if a scale is formed for featuring a group of healthy persons, it is allowed to discriminate if the individual to-be-tested persons are healthy or are suffering from some diseases relying upon the Mahalanobis distance. It may therefore become possible to prevent such an occurrence that a person suffering from some diseases is determined/discriminated to be normal and a timing for an early therapy is overlooked, or that a healthy person is judged to be suffering from some diseases and is put to an excess of precision examination. The details have been described in "Improving the Reliability of the Overall Judgement in the Health Examination by Using MTS", Quality Engineering, Vol. 7, No. 2, Yoshiko Hasegawa, et. al., April, 1999.

This invention was accomplished in an attempt to apply the MTS to the biomagnetic field measuring method. The biomagnetic field measurement simply obtains signals representing the magnetic field intensities obtained through plural magnetic sensors. How the obtained signal data be processed and used for the diagnosis has now been studied, and no definite processing method has not been proposed yet. According to this invention in an attempt to process the data, a plurality of feature parameters are calculated from the signal data of plural magnetic sensors, and a reference space is built up based on the feature parameters to thereby calculate the Mahalanobis distance. Relying upon the Mahalanobis distance, it is first determined/discriminated whether the to-be-tested person is a healthy person or a person who may be ill. As for the to-be-tested person who may be ill, the disease is estimated relying upon the feature parameters of the to-be-tested person. To estimate the disease, several disease candidates are quoted. Probabilities of diseases can be represented by quantitative numerical figures. Based on the disease candidates, the doctor conducts close examination relying upon the methods other than the one for measuring the biomagnetic field, and renders a final diagnosis. Therefore, even a doctor who is not familiar with the measurement of biomagnetic field is allowed to effectively use the biomagnetic field measuring apparatus. Further, the results of biomagnetic field measurement of the to-be-tested person can be managed by a database which can be commonly used not only in one hospital but also throughout the city, the prefecture or the country. That is, in setting the Mahalanobis distance which serves as a reference for discriminating whether the person is healthy, it is desired that the determination is rendered by making reference to the data as much as possible. This, however, can be realized by using the above database. In measuring the biomagnetic field, further, the Mahalanobis distance that serves as a reference for judging whether the person is healthy may differ depending upon the sex, age and the region where the to-be-tested person is living. By using the data of the database by taking the sex, age and the like into consideration as a reference for calculating the Mahalanobis distance for discriminating whether the person is healthy, it becomes possible to render more correct diagnosis.

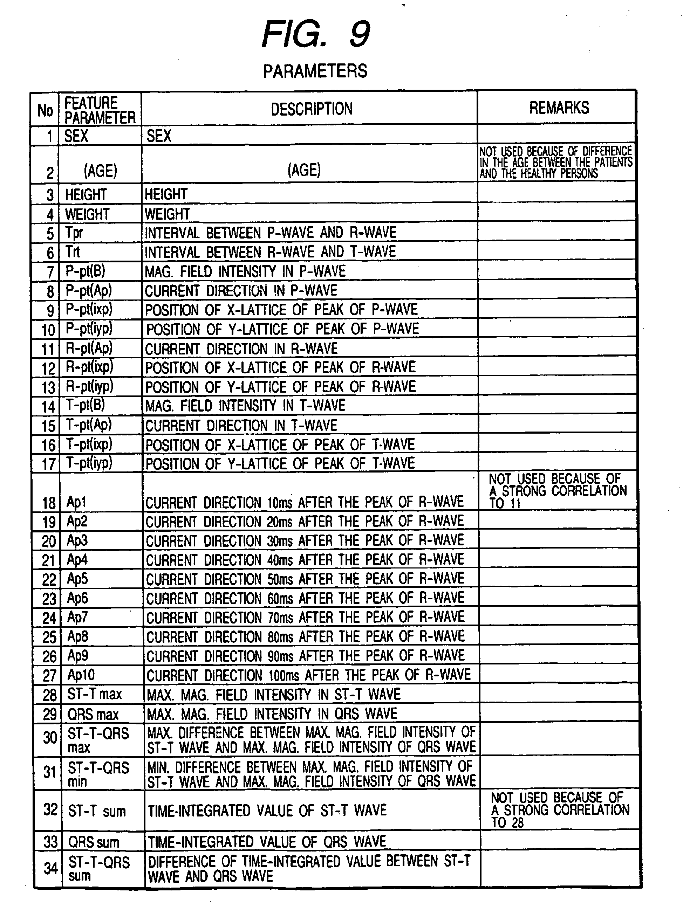

Fig. 9 illustrates feature parameters in the invention. There are 31 feature parameters including sex, height, weight, an interval of time (Tpr) giving a peak of P-wave and a peak of R-wave, an interval (Trt) between the peak of R-wave and a peak of T-wave, a magnetic field intensity (P - pt(B)) at the peak of P-wave, direction of current (P-pt(Ap)), X-position (P-pt(ixp)) and Y-position (P-pt(iyp)) of a sensor, direction of current (R - pt(Ar)) at the peak of R-wave, X-position (R -pt(ixr)) and Y-position (R- pt(iyr)) of the sensor, magnetic field intensity (T - pt(B)) at the peak of T-wave, direction of current (T - pt(At)), X-position (P - pt(ixt)) and Y-position (P - pt(iyt)) of the sensor, directions of current AP2, ---, Ap10 measured at an interval of 10 milliseconds from when 10 milliseconds have passed from the moment of peak of R-wave unitl 90 milliseconds have passed, a maximum magnetic field intensity (ST - Tmax) in an ST-T wave, a maximum magnetic field intensity (QRSmax) in a QRS wave, a maximum difference ([ST - T - QRS]max) and a minimum difference ([ST - T - QRS]min) in the maximum magnetic field intensity between the ST - T wave and the QRS wave, a time-integrated value (QRSsum) of QRS wave, and a time-integrated value difference ([ST - T - QRS]sum) between the ST - T wave and the QRS wave. The Mahalanobis distance was calculated by using the above 34 parameters. It was found that the direction of current (Ap1) 10 ms after the peak of R-wave was too strongly correlated to the direction of current (R - pt(Ap)) in the R-wave, and the time integrated value (ST - Tsum) of the ST - T wave was too strongly correlated to the maximum magnetic field intensity (ST - Tmax) in the ST - T wave, and the inverse matrix A of the correlation matrix could not be calculated, and were not, hence, used. Further, when the parameters were evaluated by the MTS method, the SN ratio in the larger-the-better characteristics was too improved when the age was used as a feature parameter due to that the ages used for forming the reference space were deviated to the twenties to the thirties. Therefore, they were not used for the evaluation. Accordingly, the Mahalanobis distance based on 31 items were used as a scale for the diagnosis.

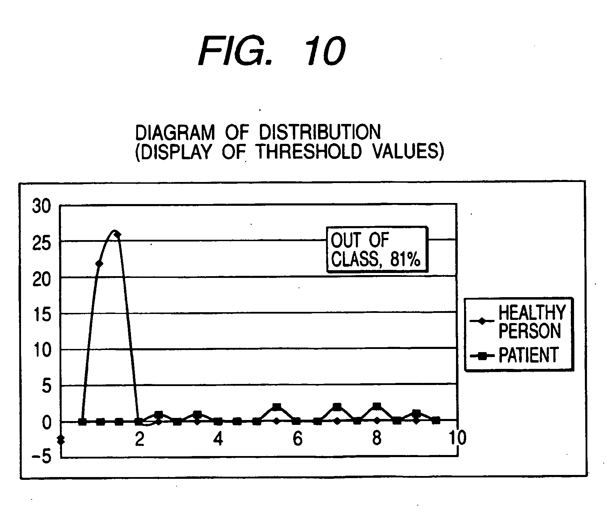

A reference space was formed from 48 healthy persons by using the above feature parameters, and the Mahalanobis distance $D^2$ was calculated for a patient 42 suffering from ischemiac cardiac disease. As shown in Fig. 10, the healthy persons and the patient suffering from cardiac disease could be discriminated maintaining a threshold value of $D^2 = 2.0$. The Mahalanobis distances were not larger than 2.0 for all healthy persons but were all $D^2 > 2.0$ for the patients suffering from the cardiac disease. Among them, 81% of data possessed the Mahalanobis distances of not smaller than 10.0 and were not within the display range of the graph. Therefore, the drawing states "out of class, 81%". When a healthy person gradually suffers the cardiac disease, the Mahalanobis distance gradually increases from the range of the healthy persons as the cardiac disease proceeds. In the initial stage of cardiac disease, therefore, it may be often difficult to make a distinction from the healthy person. However, it can be said that the Mahalanobis distance $D^2$ is effective in the diagnosis of cardiac disease if there are used suitable feature parameters and a homogeneous set of healthy persons.

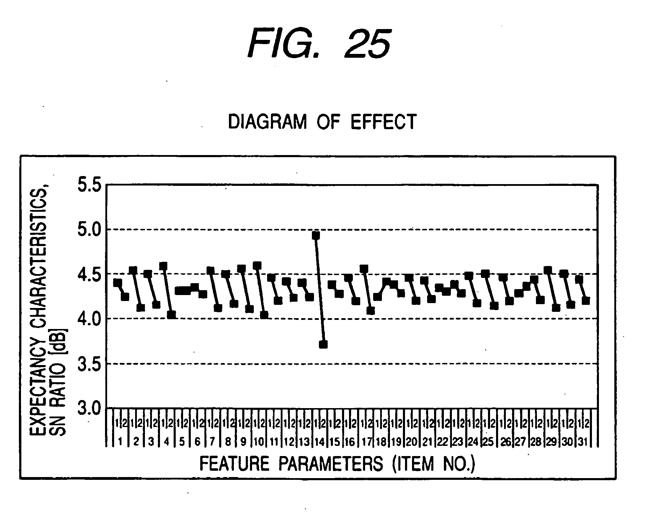

Fig. 25 is a diagram of effect of feature parameters shown in Fig. 9. The ordinate of the diagram of effect represents the SN ratio [dB] in the expectancy characteristics. The abscissa represents parameter numbers of from 1 to 31, and the numerals 1, 2 on the upper stage represent a first level and a second level. The first level is when the feature parameter is used, and the second level is when it is not used. Most parameters are descending toward the right. This demonstrates that use of the feature parameters makes it possible to discriminate the healthy persons from the patients maintaining good precision, and it can be said that the larger the degree of descend toward the right, the more effective the parameter is.

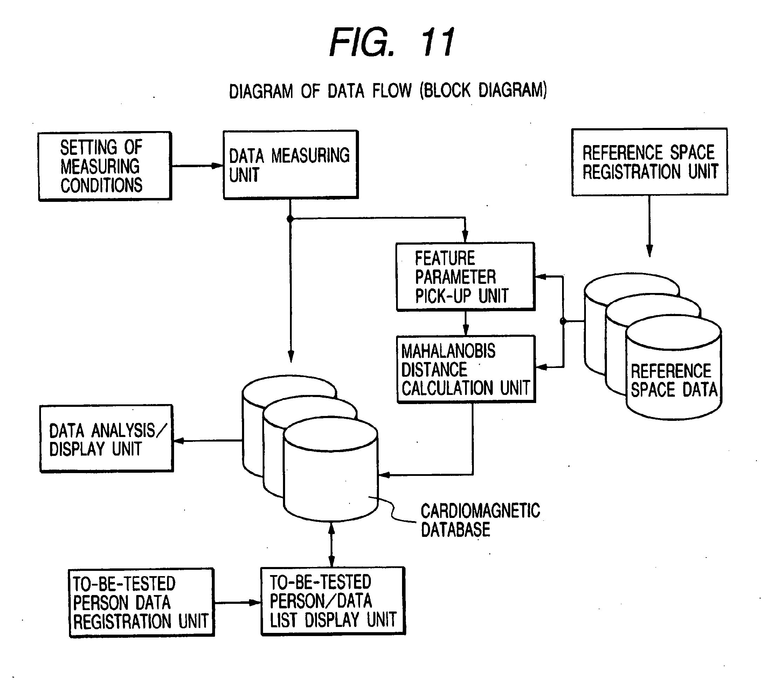

Fig. 11 is a block diagram of a software for discriminating whether the to-be-tested person is a healthy person or a patient suffering from cardiac disease relying upon the Mahalanobis distance, and illustrates the connection of functional units of a program mounted on a computer 8.

A to-be-tested person/data list display unit displays a list of data and to-be-tested persons who are to be measured and whose data are to be processed, and receives a selection from the operator. The data of the selected to-be-tested person or the data thereof are sent to a data measuring unit and to a data analysis/display unit. When the data of the to-be-tested person who is to be measured have not been registered in the cardiomagnetic database, the data of the to-be-tested person are registered to a data registration unit.

The data measuring unit controls the FLL control circuit and the AFA circuit to measure the cardiomagnetic signals from the to-be-tested person. The data of the to-be-tested person are sent from the to-be-tested person/data list display unit, and the measuring conditions are sent from the measuring condition-setting unit. The data measured in the data measuring unit are stored in the cardiomagnetic database and, at the same time, feature parameters of 31 items are calculated therefrom by the feature parameter pick-up unit, and the Mahalanobis distance is calculated by a Mahalanobis distance calculation unit. The Mahalanobis distance calculation unit makes a reference to the Mahalanobis space data. This is because, the registration and updating are accomplished by the reference space registration unit. Usually, the reference space can be calculated by using, as feature parameters, even such data related to the attribute of the to-be-tested person, such as, age, sex, occupation, race, etc. When the data of healthy persons for defining the reference space are deviated and uniformity of data cannot be maintained for the parameters, however, the healthy persons may be classified by using the attribute data of the to-be-testedpersons, and reference spaces corresponding thereto may be separately registered. The data analysis/display unit calls the data of the to-be-tested person selected by the to-be-tested person/data list display unit as well as the measured data stored in the cardiomagnetic database, and forms a variety of analytical diagrams used for the diagnosis through GUI.

A series of operations from the registration of the to-be-tested person through measuring the data of the registered to-be-tested person up to the analysis of the measured data, are carried out while looking at a screen displayed on the display 8-1. Prior to describing the series of operations, therefore, described below, first, is the layout of the displayed screen.

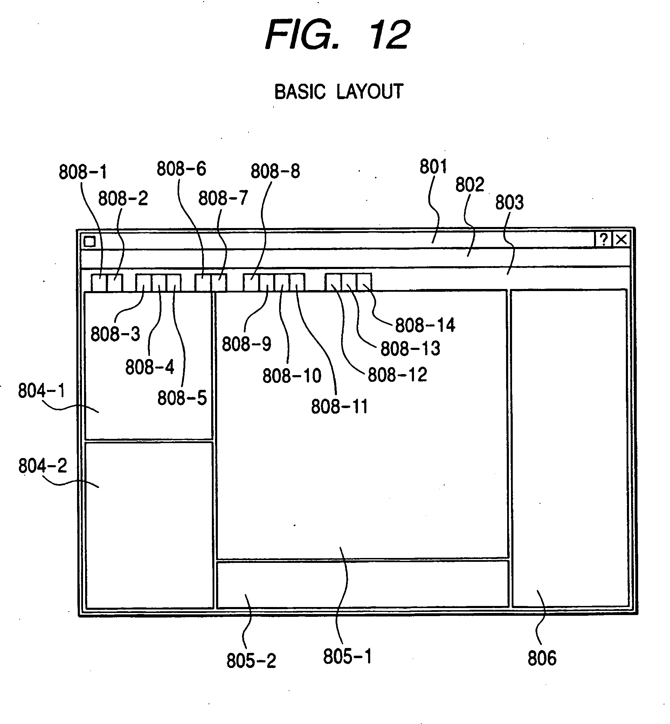

Fig. 12 illustrates a basic layout of the display screen displayed on the display 8-1 of Fig. 1. The upper part of the display screen is occupied by a title bar portion 801, a menu bar portion 802 and a tool bar portion 803 where icons are arranged, that are successively arranged from the upper side. The above portions can be considered to be display regions or areas. These arrangements are displayed in common on the display screen even for other processings, such as registering and reading the to-be-tested person, measuring the magnetic field, and processing for the analysis of measured data. This facilitates the use and makes it possible to shorten the time for measurement and processing.

The central portion of the display screen is occupied by a to-be-tested person data portion 804-1, a data portion 804-2 concerned to the analytical data, an analytical data portion 805-1 for displaying analytical data such as diagrams or waveforms, a reference waveform portion 805-2, and an operation region 806, which are arranged in order from the left toward the right.

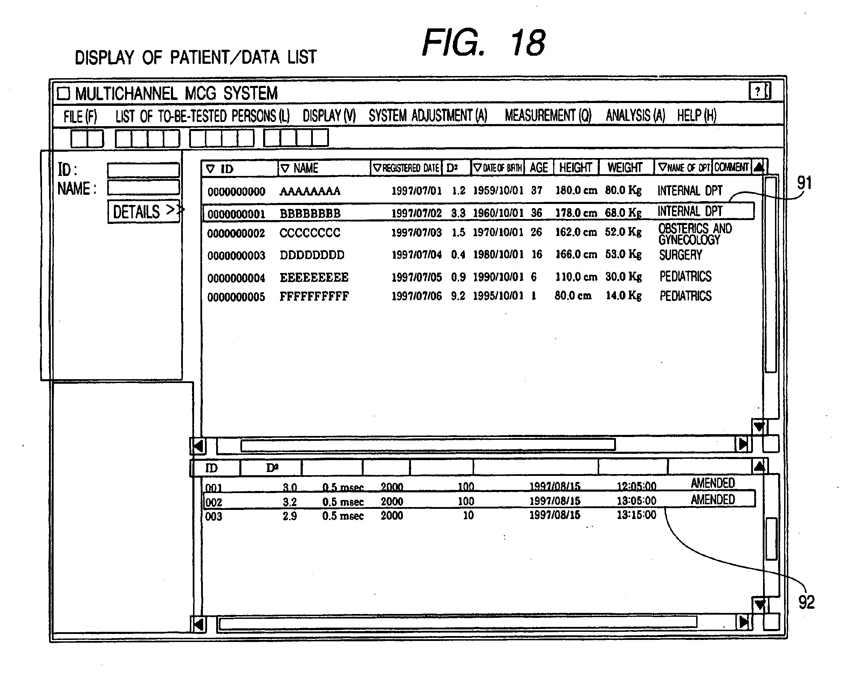

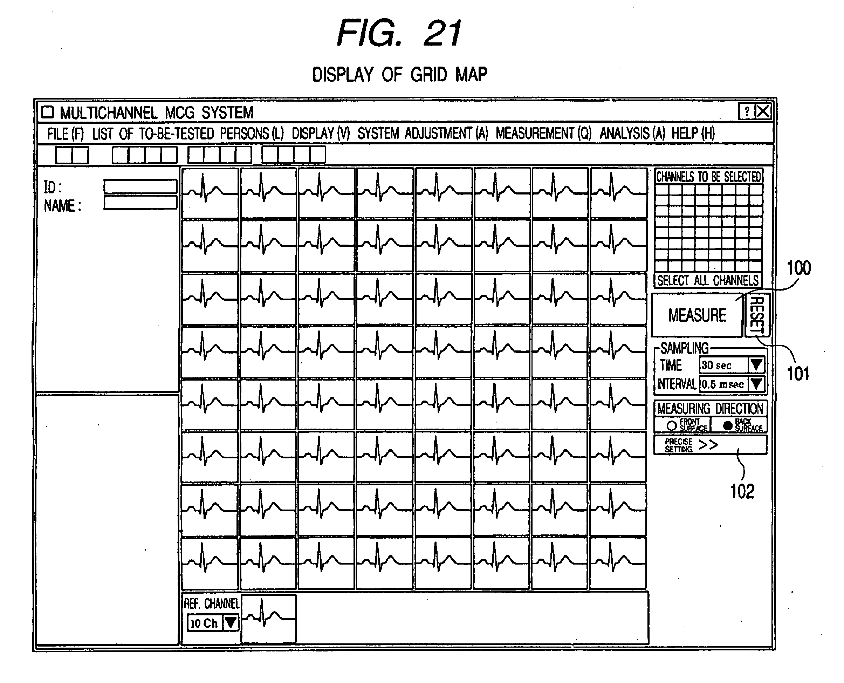

When a screen of a list of to-be-tested persons (Fig. 18) is displayed on the to-be-tested person data portion 804-1, there are displayed, at all times, the data of a to-be-tested person on which a cursor 91 is placed in the list of to-be-tested persons on the screen. When the analytical data such as diagrams or waveforms are displayed on the analytical data portion (Figs. 21 to 24), there are displayed, at all times, the data of the to-be-tested person for whom the analytical data are obtained as displayed. This makes it possible to clearly know the relationship between the analytical data that are displayed and the to-be-tested person from whom the analytical data are obtained. Similarly, when a screen of a list of to-be-tested persons (Fig. 18) is displayed on the data portion 804-2, there are displayed, at all times, the data list of a to-be-tested person selected on the screen, and there are displayed the data on which the cursor 92 is placed on the data list. When the analytical data such as diagrams or waveforms are displayed on the analytical data portion (Figs. 21 to 24), there are displayed, at all times, the analytical data as displayed. This makes it possible to clearly know the data such as time and conditions for measuring the analytical data as displayed. On the display screen of this system as described above, the to-be-tested person data portion 804-1 and the data portion 804-2 are displayed at predetermined positions (left side) of the display screen at all times like the menu bar portion 802. Therefore, the user does not have to search the data area of the to-be-tested person after every change of the display screen, but is allowed to know the data area at all times by looking at a predetermined position (left side) of the display screen.

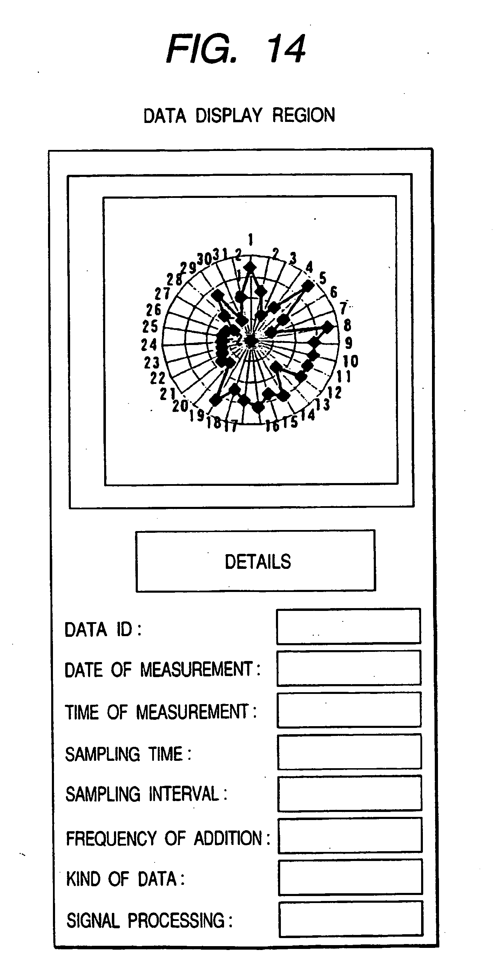

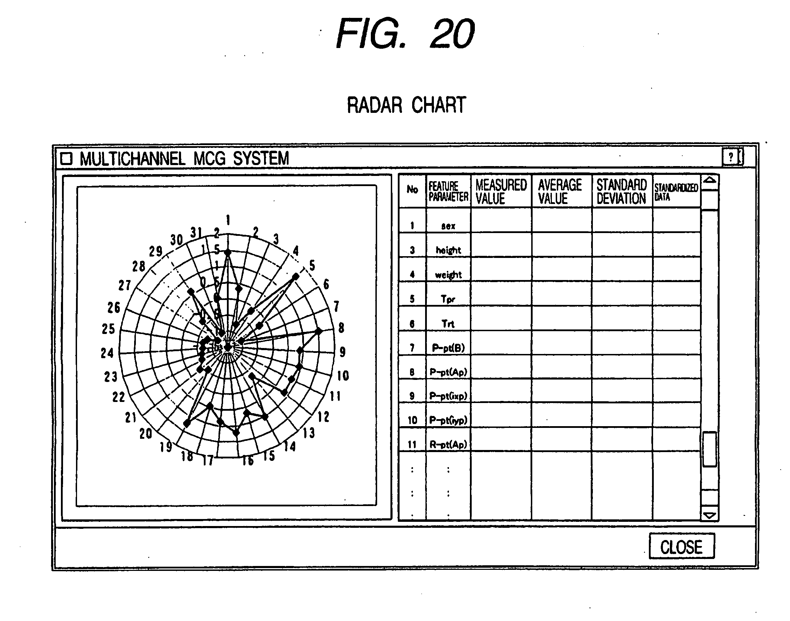

Fig. 14 illustrates the data display portion. The Mahalanobis distance $D^2$ is the attribute of the measured data and is displayed on the data portion 804-2. The radar chart shows the values of the feature parameters. This makes it possible to know any disease or a rough cause thereof (feature parameter having a peculiar value). Upon depressing the detail button on the lower side, a detailed radar chart of Fig. 20 is displayed.

The title bar portion displays the name of the frame or, concretely, the name "Multi-Channel MCG System".

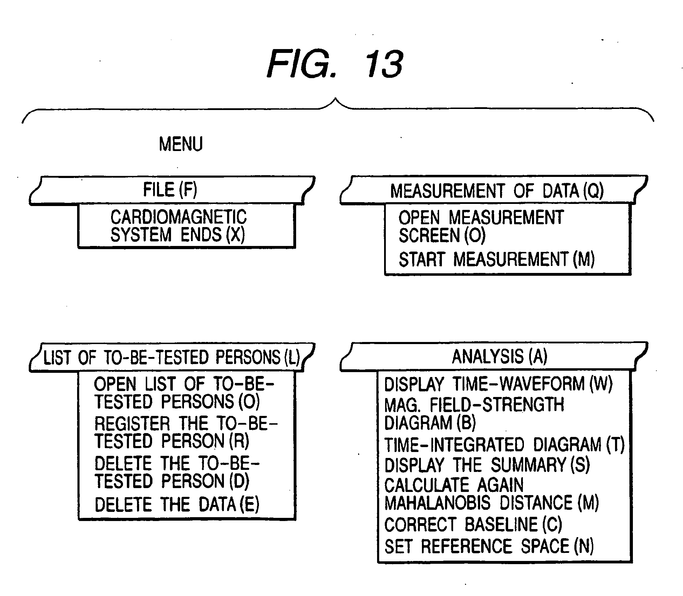

Fig. 13 illustrates the operation menu. The menu bar portion is the one for selecting the operation menu, and is capable of using such menus as "File (F)", "List of To-Be-Tested Persons (L)", "Data Measurement (Q)", and "Analysis (A)".

The contents of operation menu of Fig. 13 are displayed as a pull-down menu upon clicking the menu buttons corresponding to the contents of the menu. When the operation menu is not required, therefore, only those keywords for calling the menu are displayed in a compact manner on the menu bar portion, making it possible to widely set the display area needed for the operations, such as the analytical data portion, operation region and the like. When the operation menu is required, the keywords arranged according to the procedure of operation are selected from the menu bar portion and are displayed to instruct the operation. The keywords have been arranged in compliance with the letter arrangement (from the left to the right), and the operation can be instructed in a natural form.

The pull-down menu of "File (F)" includes an item "End of Cardiomagnetic System (X)" to end the multi-channel MCG system.

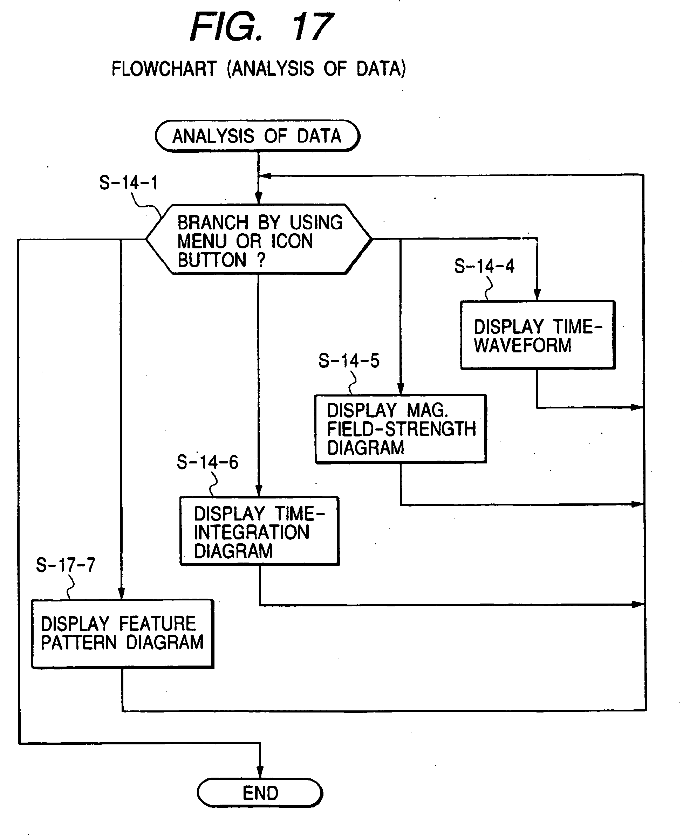

The pull-down menu of the "List of To-Be-Tested Persons (L)" includes such items as "Open the List of the To-Be-Tested Persons (O)", "Register the To-Be-Tested Person (R)", "Delete the To-Be-Tested Person (D)", and "Delete the Data (E)". When "Open the List of the To-Be-Tested Persons (O)" is selected, the screen displayed on the display unit 8-1 is changed over to the screen of the list of the to-be-tested persons (Fig. 18). When "Register the To-Be-Tested Person (R)" is selected, the dialog for registering the to-be-tested person (Fig. 17) is displayed to accept such inputs as the to-be-tested person ID, name, date of birth, height, weight, sex and comments. When "Delete the To-Be-Tested Person (D)" is selected, the data of the to-be-tested person as well as the data related to the to-be-tested person are all deleted from where the to-be-tested person data cursor 91 is placed on the list of the to-be-tested persons (Fig. 18). When "Delete the Data (E)" is selected, the data are deleted from where the data cursor 92 is placed on the data list on the screen of the list of the to-be-tested persons (Fig. 18).

The pull-down menu of "Data Measurement (Q)" in the privileged user mode includes such items as "Open the Measurement Monitor Screen (O)" and "Start the Measurement (M)". When "Open the Measurement Monitor Screen (O)" is clicked, the display on the display unit 8-1 is changed over to the measurement monitor screen shown in Fig. 21. When "Start the Measurement (M)" is clicked, the sensor state is automatically adjusted, the magnetic field is locked for all SQUID sensors, and the data are taken in under the specified conditions. After the measurement is finished, the measured data are stored in the cardiomagnetic database. At the same time, the feature parameters are picked up from the measured data and from which the Mahalanobis distance is calculated and is stored in the cardiomagnetic database as the measured data.

The pull-down menu of "Data Analysis (A)" includes "Display the Time-Waveforms (W)", "Magnetic Field-Strength Diagram (B)", "Time-Integral Diagram (T)", "Display the Summary (S)", "Re-calculation of Mahalanobis Distance (M)", "Baseline Correction (C)" and "Set the Reference Space (N)".

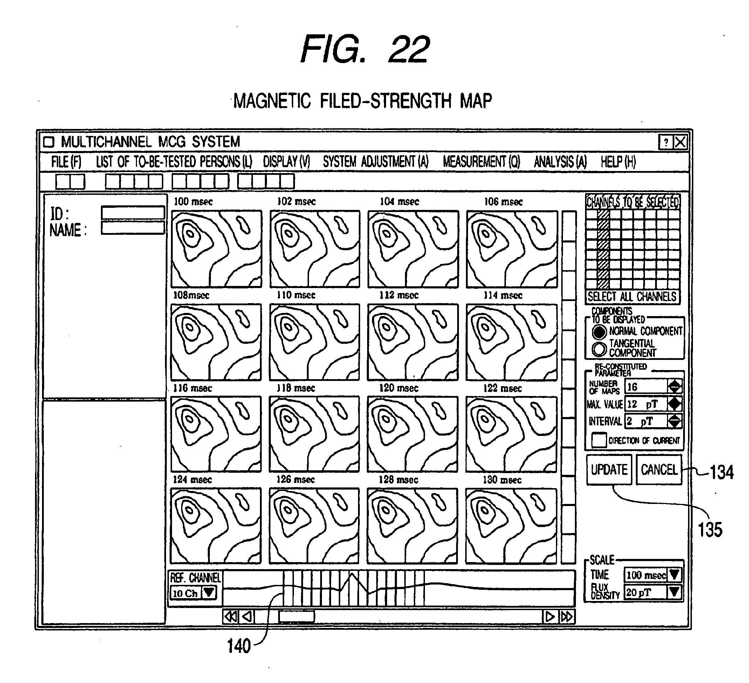

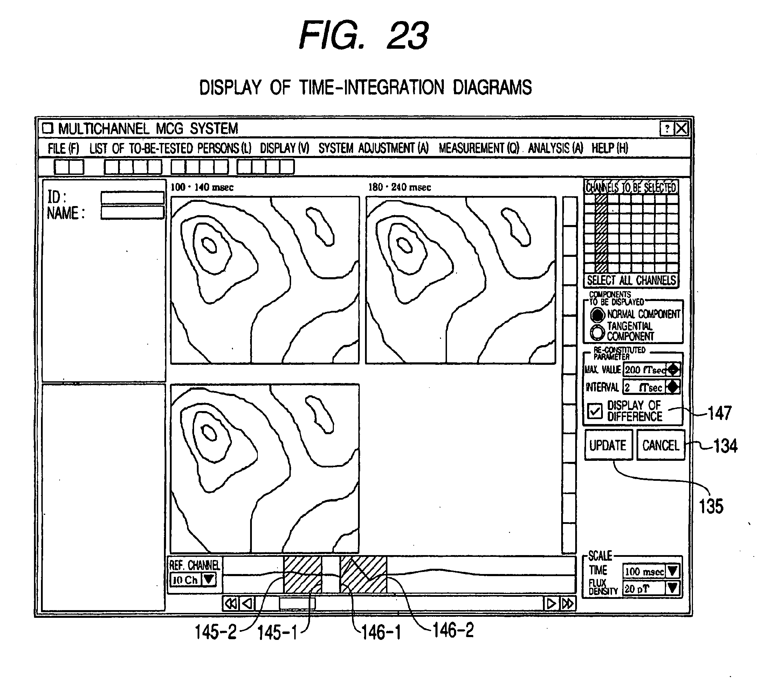

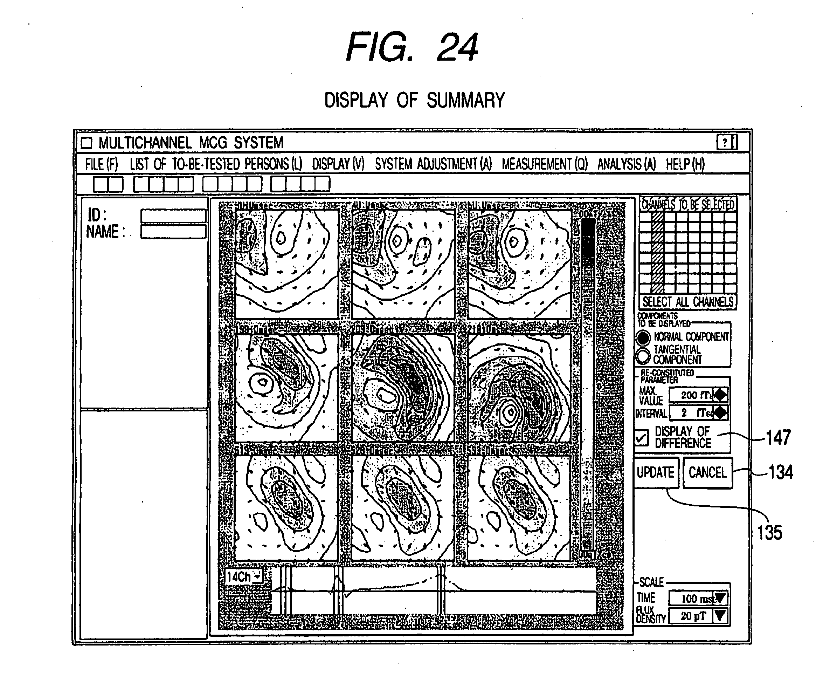

When "Display the Time-Waveforms (W)", "Magnetic Field-Strength Diagram (B)", "Time-Integral Diagram (T)" and "Display the Summary (S)" are clicked, there are displayed the screen for displaying the time-waveforms (Fig. 21), screen of magnetic field-strength diagram (Fig. 22), time-integration diagram (Fig. 23) and display the summary (Fig. 24). When "Re-calculation of Mahalanobis Distance (M)" is clicked, the Mahalanobis distance is calculated again for the data being displayed, and the value registered in the database is updated, too. When "Baseline Correction (C)" is clicked, offset is added to the baseline, i.e., the baseline is set to 0, or the level of the cardiomagnetic signals in a state where the heart is not electrophysiologically active is offset to 0 (not shown). When "Set the Reference Space (N)" is specified, there are displayed a file defining the reference space and the dialog for specifying the range of the to-be-tested persons to which it is to be applied.

In the tool bar portion 803 are arranged icon buttons related to those which are highly frequently used among those items in the pull-down menu in the operation menu.

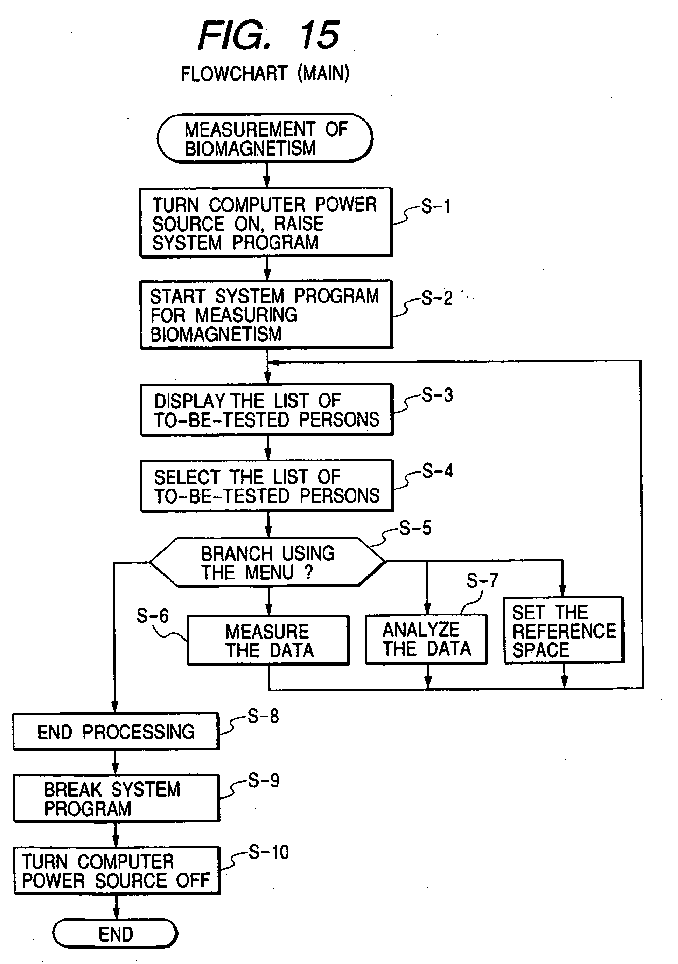

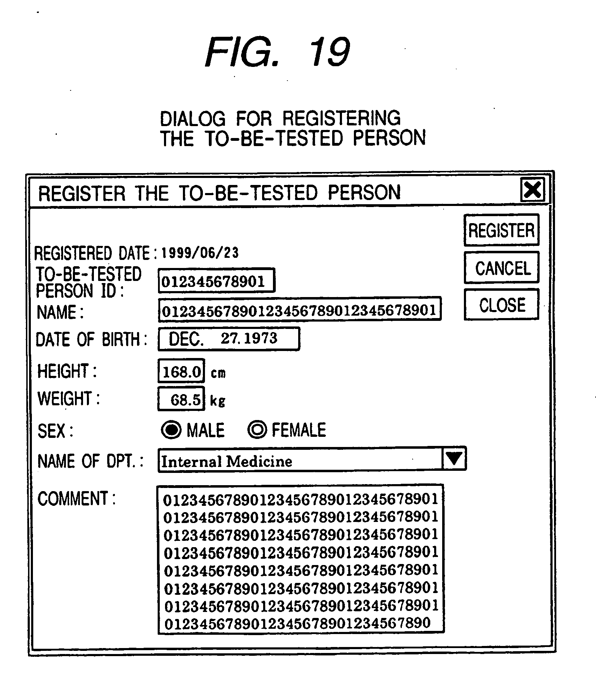

Next, described below with reference to Figs. 15 to 24 are a series of operations from the registration of the to-be-tested persons through measuring the data from the registered to-be-tested persons up to analyzing the measured data.

Fig. 15 illustrates an operation flow related to the whole biomagnetic field measuring apparatus according to the embodiment. When the power source of the computer 8 is turned on (S-1), the operation system rises, and a starter icon of a program that can be used in the computer is displayed on the display unit 8-1 (S-2). When the icon of the program of multi-channel MCG system is clicked among the icons, the list of the to-be-tested persons shown in Fig. 18 is displayed as the initial screen of the rising system (S-3).

The screen of list of the to-be-tested persons shown in Fig. 18 will now be described. The left upper portion is occupied by the to-be-tested person data portion 804, and the left lower portion is occupied by the data portion 805. Further, the list of the to-be-tested persons is displayed on the upper part and the list of data is displayed on the lower part on the whole right side. The to-be-tested person data portion displays the data of the to-be-tested person on which the cursor 91 is placed on the list of the to-be-tested persons. As the cursor 91 is moved, the displayed content is updated corresponding thereto. The list of the to-be-tested persons include ID (ID number of the to-be-tested person), name, date of registration (date on which data is registered), Mahalanobis distance $D^2$, date of birth, age, height, weight and comments (concerning the to-be-tested person.). The list of the to-be-tested persons can be scrolled by using the longitudinal scroll bar, and the items of the list of the to-be-tested persons can be scrolled by using the horizontal (transverse) scroll bar. The row of the to-be-tested person who is selected is displayed in an emphasized manner.

A list of measured data is displayed in the lower half of the screen of the list of to-be-tested persons of Fig. 18, thereby to display data attributes such as ID of data, Mahalanobis distance $D^2$, date of measurement and the like. Similarly, the list of magnetic field-strength diagrams displays the data for forming the magnetic field-strength diagrams from the measured data.

The Mahalanobis distance $D^2$ is displayed on both the list of the to-be-tested persons and the list of the measured data, the former one displaying the greatest one among the Mahalanobis distances $D^2$ in all of the measured data. In the drawing, the second patient from the above (ID 0000000001, name = BBBBBBBB) on which the cursor is placed exhibits the Mahalanobis distance $D^2 = 3.3$, which is the greatest among the Mahalanobis distances 3.0, 3.3 and 2.9 of the following three measured data.

Reverting to the whole flowchart of Fig. 15, a row of a desired to-be-tested person is selected out of the list of the to-be-tested persons on the screen of the list of to-be-tested persons at step S-4. The flow, thereafter, is branched into four by menu (S-5). According to one branch, there is selected a sub-menu "End of Cardiomagnetic System (X)" in the menu "File (F)". In this case, the end processing is conducted such as closing the window (S-8) and, then, the system is shut down (S-9). Then, the power source of the computer 8 is turned off (S-10), and everything ends.

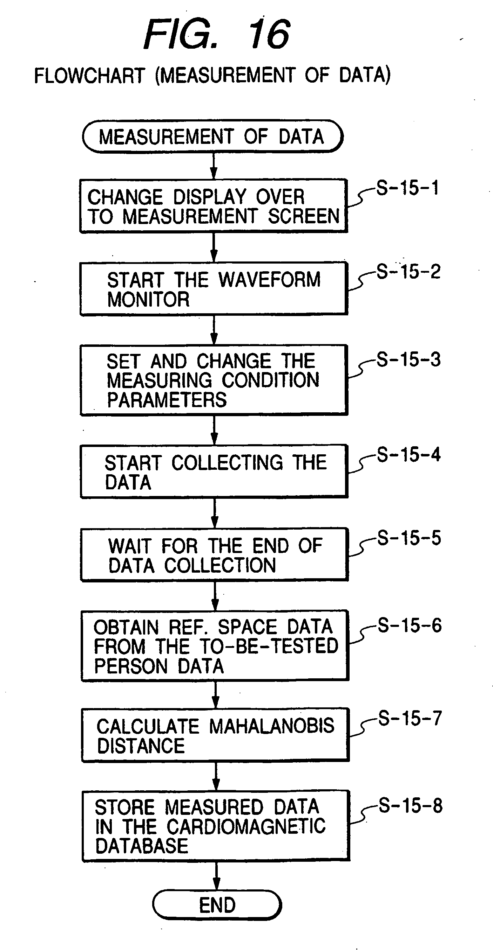

According to the remaining branch, there is conducted the measurement of data (S-6), analysis of data (S-7) or setting of reference space (S-71). The measurement of data can be executed by selecting a sub-menu "Open the Measurement Monitor Screen (O)" in the menu "Measurement of Data (Q)". The analysis of data can be executed by selecting any one of the sub-menus "Display Time-Waveforms (W)", "Magnetic Field-Strength Diagram (B)", "Time-Integral Diagram (T)" and "Display the Summary (S)" in the menu "Analysis of Data (A)". To end the steps S-6, S-7 and S-71, the routine returns back to step S-3 to display the screen for selecting the list of the to-be-tested persons. Selection of the list of the to-be-tested persons of step S-4, measurement of data of step S-6 and analysis of data of step S-7 will be described below in further detail in connection with Figs. 16 and 17.

Fig. 16 is a flowchart illustrating, in detail, the measurement of data at step S-6 in Fig. 15. First, as the initial screen of measurement, there are displayed the time-waveforms (S-15-1) by a grid map shown in Fig. 21, and a waveform monitor is started (step S-15-2). There are 8 x 8 or 64 channels, and the whole channels are selectively displayed upon clicking the "Select All Channels" button or by dragging the channel matrix from one end to the other end along the diagonal line.

The waveform monitor receives and displays the data in a periodic time (e.g., 1 second) that has been set in advance, and repeats it until the measurement button is depressed. The measurement condition parameters (step S-15-3) such as sampling time and sampling interval are set or changed, and the "Measurement" button in the operation region is depressed to start the measurement (S-15-4). As for the sampling time (measuring time) and the interval, a pull-down menu of numerals that can be selected is opened by clicking a corresponding text box marked with an inverse triangular mark, and a desired numeral can be selected out of it. The numerals that can be selected are, for example, 1 sec, 5 sec, 10 sec, 30 sec, 1 min and 2 min in the case of the time, and are, for example, 0.1 msec, 0.5 msec, 1.0 msec, 2.0 msec, 4.0 msec, 5.0 msec and 10.0 msec in the case of the interval. The time may be selected from about 1 sec to about 24 hours, as required. The "time" in the "scale" box is a time scale in a unit of milliseconds, i.e., a scale in the horizontal direction. Like selecting the sampling time and interval, any desired numeral can be selected out of the pull-down menu that is opened upon clicking the corresponding text box.

When an instruction for starting the measurement is issued for the FLL control circuit 6 and for the amplifier/filter 7, these circuits collect the data until the specified measurement ends. When the measurement ends, the computer 8-1 is interrupted and is informed of the collection of data. Thus, the control of data measurement waits for the end of collection of data (S-15-5). The measured data reads the reference space data (S-15-6), and the feature parameters defined by the reference space data are automatically taken out from the measured data (S-15-7) to calculate the Mahalanobis distance (S-15-8). The to-be-tested person data, measuring conditions, measured data and Mahalanobis distance are related to one another and are stored in the cardiomagnetic data base.

Fig. 17 is a flowchart of data analysis at step S-14 of Fig. 15. The data analysis is to display waveforms and diagrams of various kinds to obtain data necessary for the diagnosis, and is capable of selectively displaying the screens of various kinds of waveforms and diagrams upon selecting the menu of Fig. 13. That is, upon selecting the "Display the Time-Waveform (W)" of "Data Analysis (A)", the screen of time-waveform is displayed at step S-14-4. Its layout is the same as that of the time-waveform monitor shown in Fig. 21 except the operation region, and is not diagramed here. Upon selecting the "Magnetic Field-Strength diagram (B)" of "Data Analysis (A)", the magnetic field-strength diagram shown in Fig. 22 is displayed at step S-14-5. Upon selecting the "Time-Integration Diagram (T)" of "Data Analysis (A)", the screens of time-integration diagrams shown in Fig. 23 are displayed at step S-14-6. Upon selecting the "Display of Summary (S)" of "Data Analysis (A)", the screens of display the summary shown in Fig. 24 are displayed at step S-14-7.

Upon selecting the "End of Cardiomagnetic System (X)" of "File (F)", the system ends.

In the respective screens, if the icon button (808-1 to 808-15) in the tool bar is clicked, the screen of the waveform or diagram specified by the click is displayed in its place. For this purpose in Fig. 17, the branching portion is "branched by using a menu or an icon button" (S-14-1) instead of "branched by using the menu". According to this embodiment, therefore, a variety of analytical data are obtained by simply clicking the radio button in the operation region without selecting the menu of Fig. 13. Therefore, the operation time can be shortened, erroneous operation can be decreased, and operability is improved.

On the screens for analyzing the data (Figs. 21 to 24), when the display parameter is partly changed, it may often become undesirable to display the analyzed data by re-calculation. On the screen of magnetic field-strength diagram (Fig. 22), there is displayed the magnetic field-strength diagram near the R-wave. Then, in order to display the magnetic field-strength diagram to examine the magnetic field distribution in the P-wave, it is desired not only to change the display time but also to change the coloring of the color map. This is because, the magnetic field strength in the R-wave is stronger than the magnetic field strength in the P-wave. Therefore, if the coloring of the color map in the R-wave is directly applied to the magnetic field-strength diagram in the P-wave, the color tone as a whole becomes dim without accent, and the exciting portions cannot be distinctly read out. Further, if the display of image is updated every time when the parameter is updated until a desired color map is obtained, then, the response time becomes short due to the repetition of the re-drawing. Besides, meaningless images are displayed to the operator, which may lead to incorrect diagnosis. In this embodiment, this problem is solved by providing an updating button 135 and a cancel button 134 for the screen on where the data are to be analyzed. That is, the updating button 135 and the cancel button 134 are activated when the data-analyzing parameter is changed and when there is no correspondence between the content represented by the data-analyzing parameter and the content displayed on the analytical data portion 805-1. When the updating button 135 is depressed by the operator, the calculation is conducted again according to the updated data-analyzing parameter to update the display of the analytical data portion 805-1. When the cancel button 134 is depressed, further, the data-analyzing parameter is returned back to before being changed, so that the content of the data-analyzing parameter comes into agreement with the content of the analytical data portion 805-1. That is, the updating button 135 and the cancel button 134 are in an inactivated state while the content of the data-analyzing parameter is corresponded to the content displayed on the analytical data portion, and are activated when there is no correspondence between them. Therefore, when there is no correspondence between the data-analyzing parameter and the content displayed on the analytical data portion 805-1, explicit distinction is obtained depending upon the state of the updating button and the cancel button, and the analytical data are little likely to be incorrectly interpreted.

In the magnetic field-strength diagram of Fig. 22, a vertically extending narrow magnetic field strength index box 310 is arranged at the right end of the analytical data portion. The magnetic field strength index box is divided into sections of different colors. This is to distinguish the strength ranges of magnetic field represented by fringe patterns on the magnetic field-strength diagram relying upon the kinds of colors to improve visible (chromatic) recognition. That is, the central position 311 of the magnetic field strength index box 310 in the lengthwise direction is the one where the magnetic field strength is zero, and the sections over the central position are called first to sixth segments in order from the central position. Then, the first section corresponds to a magnetic field strength range of 0 to 2 pT, the second section corresponds to a magnetic field strength range of 2 to 4 pT, the third section corresponds to a magnetic field strength range of 4 to 6 pT, the fourth section corresponds to a magnetic field strength range of 6 to 8 pT, the fifth section corresponds to a magnetic field strength range of 8 to 10 pT, and the sixth section corresponds to a magnetic field strength range of 10 to 12 pT, respectively. Quite the same holds even for the sections under the central position. Here, the sections over the central position represent the magnetic field strengths in the plus direction and the sections under the central position represent the magnetic field strengths in the minus direction.

The magnetic field-strength diagrams shown in Fig. 22 are displayed by different colors depending upon the magnetic field strengths in compliance with a predetermined corresponding relationship between the magnetic field strength ranges in the magnetic field strength index boxes 310 and the colors. As for the colors, the plus side of the magnetic field strengths may be represented by hot colors, the minus side may be represented by cold colors and the central portion may be represented by yellow. This makes it possible to chromatically recognize the strengths of the magnetic field and, hence, to improve the visibility. Besides, in this embodiment, the magnetic field strength index box 310 is provided near the analytical data portion, making it possible to confirm the color of the object to be compared, i.e., to confirm the color imparted to the map in comparison with a predetermined color of the magnetic field strength index box 310 without greatly moving the eyes and, hence, to clearly judge the relationship between the levels of strength of the magnetic field and the colors.

In Fig. 22, "Number of Maps" in the box of "Re-constituted Parameter" represents the number of the magnetic field-strength diagrams that are displayed, "Maximum Value" represents the magnetic field strength corresponding to both ends of the magnetic field strength index box 310, and "Interval" represents the range of magnetic field corresponding to the length of the sections in the magnetic field strength index box 310. The values thereof can be selected by clicking a triangular button or an inversely triangular button of the corresponding text box.

In the lowermost stage of the analytical data portion, there are displayed 16 cursor lines 140 set as the waveform of the reference channel and as the number of maps, and are corresponding to 16 sheets of magnetic field-strength diagrams. The cursors are arranged maintaining an equal interval at a predetermined time. Means for setting the interval may be provided on the screen, or there may be provided means for setting the cursor lines corresponding to the plurality of magnetic field-strength diagrams one by one at irregular intervals. The positions of the cursor lines are dragged by a mouse so as to be moved toward the right and left. Here, a time for forming a first sheet of magnetic field-strength diagram may be specified by using the text box. In Fig. 22, there are displayed the magnetic field-strength diagrams of a number of 16 which are those at a moment when the cursor line is positioned on the waveform. There are further displayed the times for indicating at which moment the map is formed.

Then, in the same manner as described with reference to Fig. 22, the operator is allowed to know which range of the analytical time (width of reference waveform) is occupied by the map now displayed on the analytical data portion and to grasp at a glance which range in the analytical time is represented by the map. Therefore, the visibility is improved. Further, the range represented by the map can be easily set by simply moving the two cursors by using the mouse. If the gap among the dividing lines can be freely set, then, a dubious portion is densely indicated and other portions are coarsely indicated, making it possible to offer a variety of analytical environments to the operator.

In the time-integration diagrams of Fig. 23, three contour diagrams have been displayed on the data display region, i.e., a contour diagram by the time-integrated values in 100 to 140 milliseconds, a contour diagram by the time-integrated values in 180 to 240 milliseconds, and a contour diagram due to a difference in the two time-integrated values. The time zone of 100 to 140 milliseconds is the one in which the P-wave is generating, and the time zone of 180 to 240 milliseconds is the one in which the QRS wave is generating. The time zones are shown by the belt-like cursor in the waveform of the reference channel. In many cases, it is a practice to find the time-integration diagrams and the difference in the time-integration values in the QRS-wave and in the T-wave.

The diagram of displaying the summary of Fig. 24 is the one displaying magnetic field-strength diagrams near the peak of P-wave, near QRS-wave and near the peak of T-wave, which are considered to be important particularly for the diagnosis of cardiac disease. Displayed here are the magnetic field-strength diagrams of the peaks of P-wave, R-wave and T-wave as well as those thereof preceding and succeeding the peaks. They contain much data among the feature parameters shown in Fig. 7.

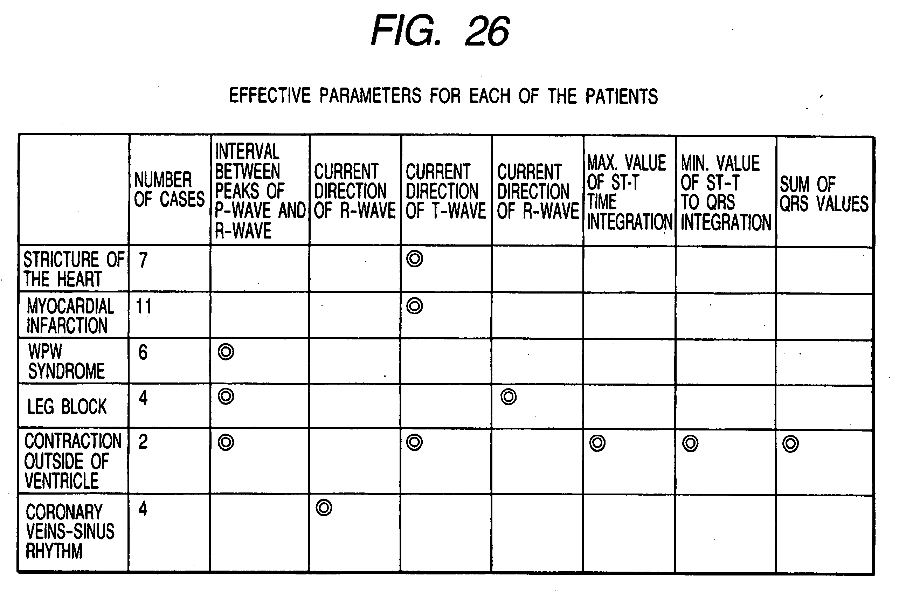

Finally, Fig. 26 shows effective parameters for each of the patients. The patients diagnosed as suffering from cardiac disease through other tests are classified for each of the names of the patients, and the diagrams of factors and effects are formed for each of the patients to clarify the feature parameters that have peculiar values. Though the number of the cases is very small, the effective parameters are remarkable for each of the patients. If relations between the diseases and the effective feature parameters are stored as data, the name of disease can be estimated from the feature parameters having peculiar values in case a large Mahalanobis distance is obtained from the data measured from the to-be-tested person.

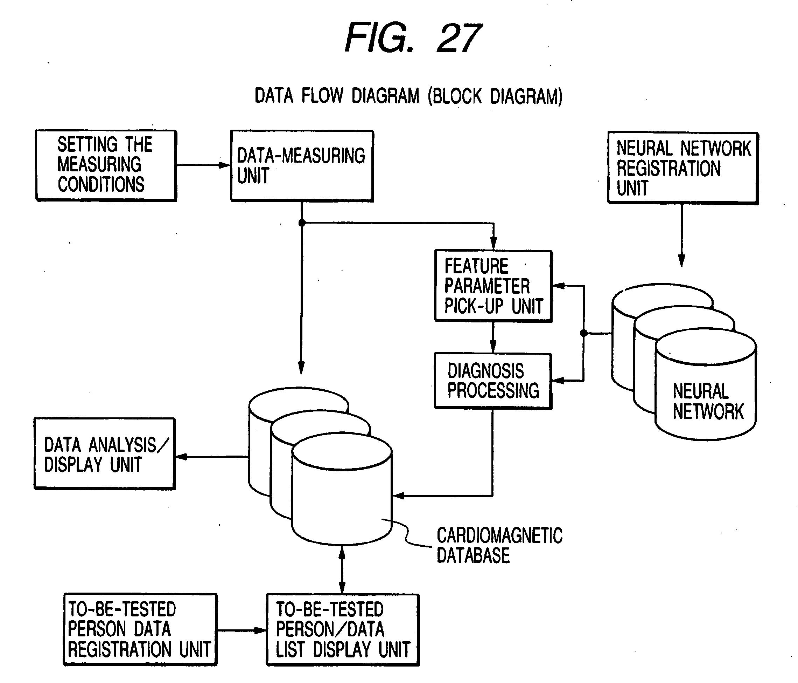

Fig. 27 is a block diagram of when a neutral network is used. As compared to the block diagram (Fig. 11) of the case of the Mahalanobis distance, the Mahalanobis distance calculation unit is replaced by a diagnosis processing unit, the reference space data is replaced by a neural network, and the reference space registration unit is replaced by a neural network registration unit.

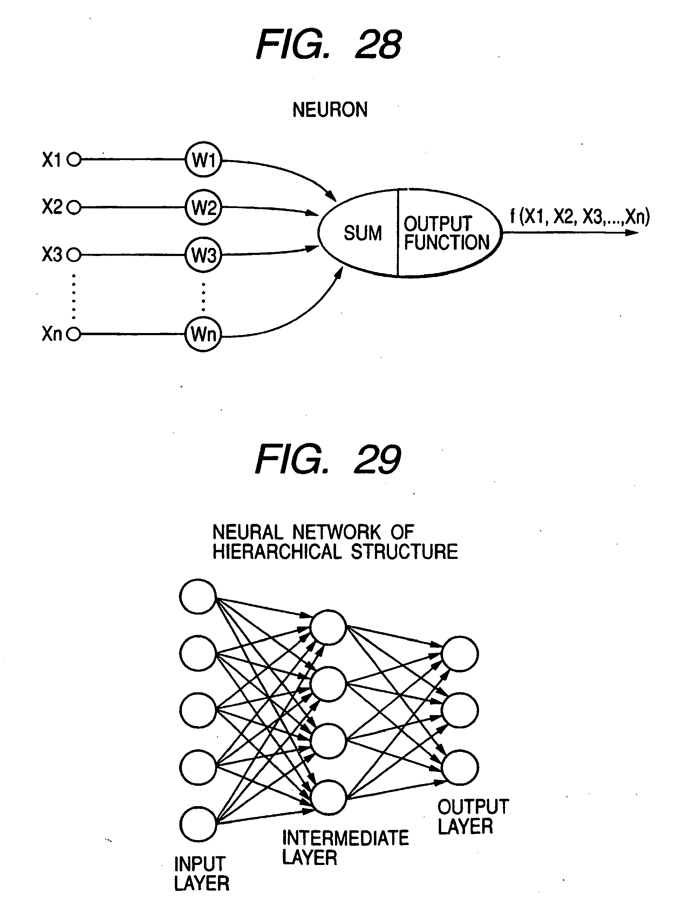

The neural network is a simulation of the data processing system of brain by using a computer. Fig. 27 illustrates a model of neurons (nerve cells) which are the data processing units, and Fig. 28 illustrates a hierarchical neural network as an example of the neural network which is a set of neurons.

It is presumed here that the neuron of Fig. 28 receives inputs $X_j$ (j = 1, ---, n) from other neurons of a number of n, the inputs being weighed by weighing coefficients $W_j$, and being input as $X_j*W_j$ to the neuron. Here, the inputs $X_j$ assume values of from 0 to 1. The weighed inputs fed to the neuron are, first, added up, and are converted into a single scalar quantity $(X_1*W_1 + X_2*W_2 + X_3*W_3 + --- + X_n*W_n)$. This sum is input, and the output of the neuron is determined depending upon the output function f thereof. As the output function, there are widely used the following threshold value function, Sigmoid function or linear function, all of which are monotonously increasing functions. A value of a range of 0 to 1 is returned back to the region of the weighed sums. By taking noise into consideration, further, the input values are often varied for the functions.

Threshold value function.

**[Mathematical 3]**

$$f(x) = \begin{cases} 1, & (x >= 0) \\ 0, & (x < 0) \end{cases}$$

Sigmoid function (Boltzmann's function as an example).

**[Mathematical 4]**

$$f(x) = 1/(1 + \exp(-kx))$$

Linear function.

**[Mathematical 5]**

$$f(x) = ax$$

The cardiac disease can be diagnosed by the neural network shown in Fig. 29, which is a combination of the neurons. The feature parameters (e.g., the ones shown in Fig. 9) picked from the signal data measured by using the cardiomagnetism measuring apparatus may be assigned to the neurons of the input layer. Here, the feature parameters have been normalized between 0 and 1. It is further presumed that probable results of diagnosis are assigned to the output layer. For example, if a healthy person and a patient suffering from cardiac disease are to be distinguished from one another, there simply exist two neurons, i.e., the neuron of the healthy person and the neuron of the patient suffering from cardiac disease. As a result of processing the diagnosis, either one of the two neurons is fired (i.e., assumes 1). If the patient suffering from cardiac disease must be diagnosed up to identifying the name of the disease, the neuron in the output layer includes those neurons that represent the healthy person, and the names of diseases such as myocardial infarction, stricture of the heart, WPW syndrome and the like. The intermediate layer consists of neurons for holding the interim results of the process of diagnosis. There may be provided a suitable number of intermediate layers as required.

According to this invention, there is provided a method of measuring biomagnetic field, which can be easily operated to favorably measure the magnetic field strengths at a plurality of measuring positions.

**Claims**

1.  A biomagnetic field measuring apparatus comprising:

   a plurality of magnetic sensors arrangeable at different positions in x-y directions in a specified area over the chest of a to-be-tested person and thereby forming a matrix of sensors for measuring magnetic field data including magnetocardiogram data generated from the heart of the to-be-tested person;
   storage means for storing the data measured by the sensors;
   feature parameter operation means for calculating a plurality of feature parameters including characteristic parameters constituting magnetic-field strength diagrams at a given moment extracted from the signal data stored in said storage means;
   Mahalanobis space-building means for building a Mahalanobis space based upon the feature parameters calculated by said operation means;
   Mahalanobis distance calculation means for calculating, relying upon the feature parameters, the Mahalanobis distance of the to-be-tested person in the Mahalanobis space built up by said Mahalanobis space building means; and

discrimination data calculation means for calculating discrimination data in order to discriminate whether the to-be-tested person is healthy or not, relying upon the data of Mahalanobis distance obtained by said Mahalanobis distance calculation means.

2.   The apparatus of claim 1, further comprising discrimination means which compares the discrimination data calculated by said discrimination data calculation means with the reference discrimination data that have been stored in advance for discriminating weather the to-be-tested person is healthy, and discriminates whether the to-be-tested person is healthy.

3.   The apparatus of claim 2, wherein said reference discrimination data are arbitrarily set by the operator.

4.   The apparatus of claim 2, further comprising a disease estimation function for selecting a candidate of disease based upon said plurality of feature parameters when it is discriminated by said discrimination means that the to-be-tested person is not healthy.

5.   The apparatus of claim 1, further comprising:

storage means for storing said feature parameters and Mahalanobis distances for each of the plurality of to-be-tested persons; and
a function for displaying at least one feature parameter and Mahalanobis distance of a to-be-tested person who is selected.

6.   The apparatus of claim 1, further comprising:

storage means for storing said feature parameters and Mahalanobis distances for each of the plurality of to-be-tested persons; and
a function for simultaneously displaying at least one feature parameter and Mahalanobis distance of a to-be-tested person who is selected, and Mahalanobis distances and feature parameters in said Mahalanobis space that is built up.

7.   The apparatus of claim 5, further comprising a function for displaying feature parameters of the to-be-tested person who is selected, an average value in a group of healthy persons defining the Mahalanobis space of the parameters, and a standard deviation thereof.

8.   The apparatus of claim 5, further comprising a function for describing feature parameters of the to-be-tested person who is selected and a standardized feature parameter.

9.   The apparatus of claim 5, further comprising a function for displaying, in an emphasized manner, the data of the to-be-tested person who has data of which the Mahalanobis distance is greater than that of the reference discrimination data that have been stored in advance in order to discriminate whether the to-be-tested person is healthy.

10.   The apparatus of claim 5, further comprising a function for displaying, in an emphasized manner, the feature parameter of which the standardized feature parameter is greater than a predetermined value when the Mahalanobis distance is greater than that of the reference discrimination data that have been stored in advance for discriminating whether the to-be-tested person is healthy.

11.   The apparatus of claim 5, further comprising a function for rearranging the data of the plurality of to-be-tested persons that are stored in order of increasing Mahalanobis distances or decreasing Mahalanobis distances.

12.   The apparatus of claim 1, further comprising:

storage means for storing a plurality of Mahalanobis space data; and
a function for selecting the space data that is used depending upon the data of the to-be-tested person.

13.   The apparatus of claim 12, further comprising a function for defining feature parameters for each of said space data.

14. The apparatus of claim 1, wherein there is further provided a function for displaying, on a screen, magnetic field-strength diagrams near P-waves, QRS-waves and T-waves among the cardiomagnetic field signal data.

15. The apparatus of claim 1, wherein the feature parameters include a current direction near an R-wave peak at regular intervals.

**Patentansprüche**

1. Messvorrichtung für biomagnetische Felder, umfassend:

   mehrere magnetische Sensoren, die an verschiedenen Positionen in x-y-Richtungen in einem vorgegebenen Bereich über der Brust einer Testperson angebracht werden können und **dadurch** eine Sensormatrix zum Messen von Magnetfeldarten, die Magnetokardiogrammdaten enthalten, die vom Herzen der Testperson erzeugt worden sind, bilden;
   Speichereinrichtung zum Speichern der von den Sensoren gemessenen Daten;
   Bearbeitungseinrichtung für Merkmalsparameter zum Berechnen mehrerer Merkmalsparameter einschließlich charakteristischer Parameter, die Magnetfeldstärkediagramme bilden, die aus den in der Speichereinrichtung gespeicherten Signaldaten zu einem vorgegebenen Zeitpunkt extrahiert worden sind;
   Mahalanobisraum-Aufbaueinrichtung zum Aufbauen eines Mahalanobisraums auf der Grundlage der von der Bearbeitungseinrichtung berechneten Merkmalsparameter;
   Mahalanobisabstand-Berechnungseinrichtung zum Berechnen, basierend auf den Merkmalsparametern, des Mahalanobisabstands der Testperson im Mahalanobisraum, der von der Mahalanobisraum-Aufbaueinrichtung aufgebaut worden ist; und
   Berechnungseinrichtung für Unterscheidungsdaten zum Berechnen von Unterscheidungsdaten, um basierend auf den von der Mahalanobisabstand-Berechnungseinrichtung erhaltenen Mahalanobisabstand-Daten zu unterscheiden, ob die Testperson gesund ist oder nicht.

2. Vorrichtung nach Anspruch 1, ferner umfassend eine Unterscheidungseinrichtung, die die von der Berechnungseinrichtung für Unterscheidungsdaten berechneten Unterscheidungsdaten mit Referenzunterscheidungsdaten vergleicht, die im voraus zum Unterscheiden, ob die Testperson gesund ist, gespeichert worden sind, und die unterscheidet, ob die Testperson gesund ist.

3. Vorrichtung nach Anspruch 2, wobei die Referenzunterscheidungsdaten vom Bediener beliebig vorgegeben sind.

4. Vorrichtung nach Anspruch 2, ferner umfassend eine Krankheitseinschätzungsfunktion zum Auswählen einer Kandidatenkrankheit auf der Grundlage der Merkmalsparameter, wenn von der Unterscheidungseinrichtung bestimmt wird, dass die Testperson nicht gesund ist.

5. Vorrichtung nach Anspruch 1, ferner umfassend:

   eine Speichereinrichtung zum Speichern der Merkmalsparameter und der Mahalanobisabstände für jede der mehreren Testpersonen und
   eine Funktion zum Anzeigen mindestens eines Merkmalsparameters und eines Mahalanobisabstands einer Testperson, die ausgewählt ist.

6. Vorrichtung nach Anspruch 1, ferner umfassend:

   eine Speichereinrichtung zum Speichern der Merkmalsparameter und Mahalanobisabstände für jede der mehreren Testpersonen und
   eine Funktion zum gleichzeitigen Anzeigen mindestens eines Merkmalsparameters und eines Mahalanobisabstands einer Testperson, die ausgewählt ist, und von Mahalanobisabständen und Merkmalsparametern im aufgebauten Mahalanobisraum.

7. Vorrichtung nach Anspruch 5, ferner umfassend eine Funktion zum Anzeigen von Merkmalsparametern der Testperson, die ausgewählt ist, von einem Durchschnittswert in einer Gruppe von den Mahalanobisraum der Parameter definierenden gesunden Personen und von einer Standardabweichung davon.

8. Vorrichtung nach Anspruch 5, ferner umfassend eine Funktion zum Beschreiben von Merkmalsparametern der Testperson, die ausgewählt ist, und von einem standardisierten Merkmalsparameter.

9. Vorrichtung nach Anspruch 5, ferner umfassend eine Funktion zum hervorgehobenen Anzeigen der Daten der Testperson, die Daten aufweist, deren Mahalanobisabstand größer als der der Referenzunterscheidungsdaten ist, die im voraus zum Unterscheiden, ob die Testperson gesund ist, gespeichert worden sind.

10. Vorrichtung nach Anspruch 5, ferner umfassend eine Funktion zum hervorgehobenen Anzeigen des Merkmalsparameters, dessen standardisierter Merkmalsparameter größer als ein vorgegebener Wert ist, wenn der Mahalanobisabstand größer als der der Referenzunterscheidungsdaten ist, die im voraus zum Unterscheiden, ob die Testperson gesund ist, gespeichert worden sind.

11. Vorrichtung nach Anspruch 5, ferner umfassend eine Funktion zum Umordnen der Daten der mehreren Testpersonen, die gespeichert worden sind, in der Reihenfolge aufsteigender Mahalanobisabstände oder absteigender Mahalanobisabstände.

12. Vorrichtung nach Anspruch 1, ferner umfassend:

eine Speichereinrichtung zum Speichern mehrerer Mahalanobisraumdaten und
eine Funktion zum Auswählen der Raumdaten, die in Abhängigkeit von den Daten der Testperson verwendet werden.

13. Vorrichtung nach Anspruch 12, ferner umfassend eine Funktion zum Definieren von Merkmalsparametern für alle solchen Raumdaten.

14. Vorrichtung nach Anspruch 1, wobei ferner eine Funktion zum Anzeigen auf einem Bildschirm von Magnetfeldstärkediagrammen in der Nähe von P-, QRS- und T-Wellen in den Signaldaten des kardiomagnetischen Felds vorgesehen ist.

15. Vorrichtung nach Anspruch 1, wobei die Merkmalsparameter eine Stromrichtung in der Nähe einer R-Wellenspitze in regelmäßigen Intervallen enthalten.

**Revendications**

1. Dispositif de mesure de champ biomagnétique comportant :

une pluralité de capteurs magnétiques pouvant être agencés à différentes positions dans des directions x-y dans une zone spécifiée sur la poitrine d'une personne à tester et formant ainsi une matrice de capteurs pour mesurer des données de champ magnétique incluant des données de magnétocardiogramme générées par le coeur de la personne à tester,
des moyens de mémorisation pour mémoriser les données mesurées par les capteurs,
des moyens d'exploitation de paramètres d'entités pour calculer une pluralité de paramètres d'entités incluant des paramètres caractéristiques constituant des diagrammes d'intensité de champ magnétique à un moment donné, extraits des données de signal mémorisées dans lesdits moyens de mémorisation,
des moyens de construction d'espace de Mahalanobis pour construire un espace de Mahalanobis basé sur les paramètres d'entités calculés par lesdits moyens d'exploitation,
des moyens de calcul de distance de Mahalanobis pour calculer, en s'appuyant sur les paramètres d'entités, la distance de Mahalanobis de la personne à tester dans l'espace de Mahalanobis construit par lesdits moyens de construction d'espace de Mahalanobis, et
des moyens de calcul de données de discrimination pour calculer des données de discrimination afin de discriminer si oui ou non la personne à tester est en bonne santé, en s'appuyant sur les données de la distance de Mahalanobis obtenue par lesdits moyens de calcul de distance de Mahalanobis.

2. Dispositif selon la revendication 1, comportant en outre des moyens de discrimination qui comparent les données de discrimination calculées par lesdits moyens de calcul de données de discrimination aux données de discrimination de référence qui ont été mémorisées à l'avance pour discriminer si la personne à tester est en bonne santé, et discrimine si la personne à tester est en bonne santé.

**3.** Dispositif selon la revendication 2, dans lequel lesdites données de discrimination de référence sont définies de manière arbitraire par l'opérateur.

**4.** Dispositif selon la revendication 2, comportant en outre une fonction d'estimation de maladie pour sélectionner un candidat à la maladie sur la base de ladite pluralité de paramètres d'entités lorsque lesdits moyens de discrimination discriminent que la personne à tester n'est pas en bonne santé.

**5.** Dispositif selon la revendication 1, comportant en outre :

des moyens de mémorisation pour mémoriser lesdits paramètres d'entités et lesdites distances de Mahalanobis pour chaque personne parmi la pluralité de personnes à tester, et
une fonction pour afficher au moins un paramètre d'entité et une distance de Mahalanobis d'une personne à tester qui est sélectionnée.

**6.** Dispositif selon la revendication 1, comportant en outre :

des moyens de mémorisation pour mémoriser lesdits paramètres d'entités et lesdites distances de Mahalanobis pour chaque personne parmi la pluralité de personnes à tester, et
une fonction pour afficher simultanément au moins un paramètre d'entité et une distance de Mahalanobis d'une personne à tester qui est sélectionnée, et des distances de Mahalanobis et des paramètres d'entités dans ledit espace de Mahalanobis qui est construit.

**7.** Dispositif selon la revendication 5, comportant en outre une fonction pour afficher des paramètres d'entités de la personne à tester qui est sélectionnée, une valeur moyenne dans un groupe de personnes en bonne santé définissant l'espace de Mahalanobis des paramètres, et son écart type.

**8.** Dispositif selon la revendication 5, comportant en outre une fonction pour décrire des paramètres d'entités de la personne à tester qui est sélectionnée et un paramètre d'entité standardisé.

**9.** Dispositif selon la revendication 5, comportant en outre une fonction pour afficher, d'une manière accentuée, les données de la personne à tester qui a des données dont la distance de Mahalanobis est supérieure à celle des données de discrimination de référence qui ont été mémorisées à l'avance afin de discriminer si la personne à tester est en bonne santé.

**10.** Dispositif selon la revendication 5, comportant en outre une fonction pour afficher, d'une manière accentuée, le paramètre d'entité dont le paramètre d'entité standardisé est supérieur à une valeur prédéterminée lorsque la distance de Mahalanobis est supérieure à celle des données de discrimination de référence qui ont été mémorisées à l'avance pour discriminer si la personne à tester est en bonne santé.

**11.** Dispositif selon la revendication 5, comportant en outre une fonction pour réagencer les données de la pluralité de personnes à tester qui sont mémorisées afin d'accroître les distances de Mahalanobis ou de réduire les distances de Mahalanobis.

**12.** Dispositif selon la revendication 1, comportant en outre :

des moyens de mémorisation pour mémoriser une pluralité de données d'espace de Mahalanobis, et
une fonction pour sélectionner les données d'espace qui sont utilisées en fonction des données de la personne à tester.

**13.** Dispositif selon la revendication 12, comportant en outre une fonction pour définir des paramètres d'entités de chacune desdites données d'espace.

**14.** Dispositif selon la revendication 1, dans lequel on fournit en outre une fonction pour afficher, sur un écran, des diagrammes d'intensité de champ magnétique à proximité d'ondes P, d'ondes QRS, et d'ondes T parmi les données de signal de champ cardiomagnétique.

**15.** Dispositif selon la revendication 1, dans lequel les paramètres d'entités incluent une direction de courant près d'un pic d'onde R à des intervalles réguliers.

# FIG. 1

## SYSTEM CONSTITUTION

# FIG. 2

## SENSOR SYSTEM FOR MEASUREMENT

# FIG. 3

SENSOR UNIT

(a) FOR MEASURING
NORMAL COMPONENT

(b) FOR MEASURING
TANGENTIAL COMPONENT

# FIG. 4

RANGE OF
MEASUREMENT

27-8

27-1

30

20-1

20-8

Y

X

# FIG. 5

AN EXAMPLE OF
MAGNETOCARDIOGRAM

0    100    200    300    400    500    600    700
msec

P            QRS        ST
                        ST-T

# FIG. 6

## WHEN DIAGNOSING A HEALTHY PERSON

| | MAG. FIELD-STRENGTH DIAGRAM OF NORMAL COMPONENTS | MAG. FIELD-STRENGTH DIAGRAM OF TANGENTIAL COMPONENTS |
|---|---|---|
| P-WAVE | | |
| S-WAVE | | |
| ST-WAVE | | |

# FIG. 7

## PATIENT SUFFERING FROM MYOCARDIAL INFARCTION

# FIG. 8

## PATIENT SUFFERING FROM WPW SYNDROME

# FIG. 9

PARAMETERS

| No | FEATURE PARAMETER | DESCRIPTION | REMARKS |
|---|---|---|---|
| 1 | SEX | SEX | |
| 2 | (AGE) | (AGE) | NOT USED BECAUSE OF DIFFERENCE IN THE AGE BETWEEN THE PATIENTS AND THE HEALTHY PERSONS |
| 3 | HEIGHT | HEIGHT | |
| 4 | WEIGHT | WEIGHT | |
| 5 | Tpr | INTERVAL BETWEEN P-WAVE AND R-WAVE | |
| 6 | Trt | INTERVAL BETWEEN R-WAVE AND T-WAVE | |
| 7 | P-pt(B) | MAG. FIELD INTENSITY IN P-WAVE | |
| 8 | P-pt(Ap) | CURRENT DIRECTION IN P-WAVE | |
| 9 | P-pt(ixp) | POSITION OF X-LATTICE OF PEAK OF P-WAVE | |
| 10 | P-pt(iyp) | POSITION OF Y-LATTICE OF PEAK OF P-WAVE | |
| 11 | R-pt(Ap) | CURRENT DIRECTION IN R-WAVE | |
| 12 | R-pt(ixp) | POSITION OF X-LATTICE OF PEAK OF R-WAVE | |
| 13 | R-pt(iyp) | POSITION OF Y-LATTICE OF PEAK OF R-WAVE | |
| 14 | T-pt(B) | MAG. FIELD INTENSITY IN T-WAVE | |
| 15 | T-pt(Ap) | CURRENT DIRECTION IN T-WAVE | |
| 16 | T-pt(ixp) | POSITION OF X-LATTICE OF PEAK OF T-WAVE | |
| 17 | T-pt(iyp) | POSITION OF Y-LATTICE OF PEAK OF T-WAVE | |
| 18 | Ap1 | CURRENT DIRECTION 10ms AFTER THE PEAK OF R-WAVE | NOT USED BECAUSE OF A STRONG CORRELATION TO 11 |
| 19 | Ap2 | CURRENT DIRECTION 20ms AFTER THE PEAK OF R-WAVE | |
| 20 | Ap3 | CURRENT DIRECTION 30ms AFTER THE PEAK OF R-WAVE | |
| 21 | Ap4 | CURRENT DIRECTION 40ms AFTER THE PEAK OF R-WAVE | |
| 22 | Ap5 | CURRENT DIRECTION 50ms AFTER THE PEAK OF R-WAVE | |
| 23 | Ap6 | CURRENT DIRECTION 60ms AFTER THE PEAK OF R-WAVE | |
| 24 | Ap7 | CURRENT DIRECTION 70ms AFTER THE PEAK OF R-WAVE | |
| 25 | Ap8 | CURRENT DIRECTION 80ms AFTER THE PEAK OF R-WAVE | |
| 26 | Ap9 | CURRENT DIRECTION 90ms AFTER THE PEAK OF R-WAVE | |
| 27 | Ap10 | CURRENT DIRECTION 100ms AFTER THE PEAK OF R-WAVE | |
| 28 | ST-T max | MAX. MAG. FIELD INTENSITY IN ST-T WAVE | |
| 29 | QRS max | MAX. MAG. FIELD INTENSITY IN QRS WAVE | |
| 30 | ST-T-QRS max | MAX. DIFFERENCE BETWEEN MAX. MAG. FIELD INTENSITY OF ST-T WAVE AND MAX. MAG. FIELD INTENSITY OF QRS WAVE | |
| 31 | ST-T-QRS min | MIN. DIFFERENCE BETWEEN MAX. MAG. FIELD INTENSITY OF ST-T WAVE AND MAX. MAG. FIELD INTENSITY OF QRS WAVE | |
| 32 | ST-T sum | TIME-INTEGRATED VALUE OF ST-T WAVE | NOT USED BECAUSE OF A STRONG CORRELATION TO 28 |
| 33 | QRS sum | TIME-INTEGRATED VALUE OF QRS WAVE | |
| 34 | ST-T-QRS sum | DIFFERENCE OF TIME-INTEGRATED VALUE BETWEEN ST-T WAVE AND QRS WAVE | |

# FIG. 10

DIAGRAM OF DISTRIBUTION
(DISPLAY OF THRESHOLD VALUES)

# FIG. 11

DIAGRAM OF DATA FLOW (BLOCK DIAGRAM)

# FIG. 12

## BASIC LAYOUT

# FIG. 13

MENU

**FILE (F)**

CARDIOMAGNETIC
SYSTEM ENDS (X)

**MEASUREMENT OF DATA (Q)**

OPEN MEASUREMENT
SCREEN (O)

START MEASUREMENT (M)

**LIST OF TO-BE-TESTED PERSONS (L)**

OPEN LIST OF TO-BE-
TESTED PERSONS (O)

REGISTER THE TO-BE-
TESTED PERSON (R)

DELETE THE TO-BE-
TESTED PERSON (D)

DELETE THE DATA (E)

**ANALYSIS (A)**

DISPLAY TIME-WAVEFORM (W)

MAG. FIELD-STRENGTH
DIAGRAM (B)

TIME-INTEGRATED DIAGRAM (T)

DISPLAY THE SUMMARY (S)

CALCULATE AGAIN
MAHALANOBIS DISTANCE (M)

CORRECT BASELINE (C)

SET REFERENCE SPACE (N)

# FIG. 14

DATA DISPLAY REGION

DETAILS

DATA ID :

DATE OF MEASUREMENT :

TIME OF MEASUREMENT :

SAMPLING TIME :

SAMPLING INTERVAL :

FREQUENCY OF ADDITION :

KIND OF DATA :

SIGNAL PROCESSING :

# FIG. 15

FLOWCHART (MAIN)

```
   ┌──────────────────────┐
   │   MEASUREMENT OF     │
   │   BIOMAGNETISM       │
   └──────────┬───────────┘
              │
   ┌──────────▼───────────┐
   │ TURN COMPUTER POWER  │  S-1
   │ SOURCE ON, RAISE     │
   │ SYSTEM PROGRAM       │
   └──────────┬───────────┘
              │
   ┌──────────▼───────────┐
   │ START SYSTEM PROGRAM │  S-2
   │ FOR MEASURING        │
   │ BIOMAGNETISM         │
   └──────────┬───────────┘
              │
   ┌──────────▼───────────┐
   │ DISPLAY THE LIST OF  │  S-3
   │ TO-BE-TESTED PERSONS │
   └──────────┬───────────┘
              │
   ┌──────────▼───────────┐
   │ SELECT THE LIST OF   │  S-4
   │ TO-BE-TESTED PERSONS │
   └──────────┬───────────┘
              │
        ┌─────▼─────┐  S-5
        │  BRANCH USING  │
        │  THE MENU ?    │
        └─────┬─────┘
              │
   MEASURE THE DATA  S-6
   ANALYZE THE DATA  S-7
   SET THE REFERENCE SPACE

   END PROCESSING  S-8

   BREAK SYSTEM PROGRAM  S-9

   TURN COMPUTER POWER SOURCE OFF  S-10

   END
```

# FIG. 16

FLOWCHART (MEASUREMENT OF DATA)

```
        ( MEASUREMENT OF DATA )
                  │
                  ▼
   ┌──────────────────────────┐
   │ CHANGE DISPLAY OVER       │  S-15-1
   │ TO MEASUREMENT SCREEN     │
   └──────────────────────────┘
                  │
                  ▼
   ┌──────────────────────────┐
   │ START THE WAVEFORM        │  S-15-2
   │ MONITOR                   │
   └──────────────────────────┘
                  │
                  ▼
   ┌──────────────────────────┐
   │ SET AND CHANGE THE        │  S-15-3
   │ MEASURING CONDITION       │
   │ PARAMETERS                │
   └──────────────────────────┘
                  │
                  ▼
   ┌──────────────────────────┐
   │ START COLLECTING THE      │  S-15-4
   │ DATA                      │
   └──────────────────────────┘
                  │
                  ▼
   ┌──────────────────────────┐
   │ WAIT FOR THE END OF       │  S-15-5
   │ DATA COLLECTION           │
   └──────────────────────────┘
                  │
                  ▼
   ┌──────────────────────────┐
   │ OBTAIN REF. SPACE DATA    │  S-15-6
   │ FROM THE TO-BE-TESTED     │
   │ PERSON DATA               │
   └──────────────────────────┘
                  │
                  ▼
   ┌──────────────────────────┐
   │ CALCULATE MAHALANOBIS     │  S-15-7
   │ DISTANCE                  │
   └──────────────────────────┘
                  │
                  ▼
   ┌──────────────────────────┐
   │ STORE MEASURED DATA       │  S-15-8
   │ IN THE CARDIOMAGNETIC     │
   │ DATABASE                  │
   └──────────────────────────┘
                  │
                  ▼
             (   END   )
```

# FIG. 17

FLOWCHART (ANALYSIS OF DATA)

DISPLAY OF PATIENT/DATA LIST

# FIG. 18

EP 1 312 304 B1

☐ MULTICHANNEL MCG SYSTEM                                                           [?]

FILE (F)    LIST OF TO-BE-TESTED PERSONS (L)    DISPLAY (V)    SYSTEM ADJUSTMENT (A)    MEASUREMENT (Q)    ANALYSIS (A)    HELP (H)

ID :
NAME :

DETAILS >

| ▽ ID | ▽ NAME | ▽REGISTERED DATE | D² | ▽DATE OF BRTH | AGE | HEIGHT | WEIGHT | ▽NAME OF DPT | COMMENT |
|---|---|---|---|---|---|---|---|---|---|
| 0000000000 | AAAAAAAA | 1997/07/01 | 1.2 | 1959/10/01 | 37 | 180.0 cm | 80.0 Kg | INTERNAL DPT | |
| 0000000001 | BBBBBBBB | 1997/07/02 | 3.3 | 1960/10/01 | 36 | 178.0 cm | 68.0 Kg | INTERNAL DPT | |
| 0000000002 | CCCCCCCC | 1997/07/03 | 1.5 | 1970/10/01 | 26 | 162.0 cm | 52.0 Kg | OBSTERICS AND GYNECOLOGY | |
| 0000000003 | DDDDDDDD | 1997/07/04 | 0.4 | 1980/10/01 | 16 | 166.0 cm | 53.0 Kg | SURGERY | |
| 0000000004 | EEEEEEEEE | 1997/07/05 | 0.9 | 1990/10/01 | 6 | 110.0 cm | 30.0 Kg | PEDIATRICS | |
| 0000000005 | FFFFFFFFFF | 1997/07/06 | 9.2 | 1995/10/01 | 1 | 80.0 cm | 14.0 Kg | PEDIATRICS | |

91

| ID | D² | | | | | | |
|---|---|---|---|---|---|---|---|
| 001 | 3.0 | 0.5 msec | 2000 | 100 | 1997/08/15 | 12:05:00 | AMENDED |
| 002 | 3.2 | 0.5 msec | 2000 | 100 | 1997/08/15 | 13:05:00 | AMENDED |
| 003 | 2.9 | 0.5 msec | 2000 | 10 | 1997/08/15 | 13:15:00 | |

92

# FIG. 19

DIALOG FOR REGISTERING
THE TO-BE-TESTED PERSON

REGISTER THE TO-BE-TESTED PERSON  ☒

REGISTER

CANCEL

CLOSE

REGISTERED DATE : 1999/06/23

TO-BE-TESTED
PERSON ID :  012345678901

NAME :  012345678901234567890123456789012

DATE OF BIRTH :  DEC.  27.1973

HEIGHT :  168.0 cm

WEIGHT :  68.5 kg

SEX :  ◉ MALE  ◎ FEMALE

NAME OF DPT. :  Internal Medicine  ▼

COMMENT :
012345678901234567890123456789012
012345678901234567890123456789012
012345678901234567890123456789012
012345678901234567890123456789012
012345678901234567890123456789012
012345678901234567890123456789012
012345678901234567890123456789012
01234567890123456789012345 67890

# FIG. 20

## RADAR CHART

| No | FEATURE PARAMETER | MEASURED VALUE | AVERAGE VALUE | STANDARD DEVIATION | STANDARDIZED DATA |
|---|---|---|---|---|---|
| 1 | sex | | | | |
| 3 | height | | | | |
| 4 | weight | | | | |
| 5 | Tpr | | | | |
| 6 | Trt | | | | |
| 7 | P-pt(B) | | | | |
| 8 | P-pt(Ap) | | | | |
| 9 | P-pt(ixp) | | | | |
| 10 | P-pt(iyp) | | | | |
| 11 | R-pt(Ap) | | | | |
| : | : | | | | |
| : | : | | | | |

CLOSE

EP 1 312 304 B1

# FIG. 21

## DISPLAY OF GRID MAP

□ MULTICHANNEL MCG SYSTEM [?] [X]

FILE (F)  LIST OF TO-BE-TESTED PERSONS (L)  DISPLAY (V)  SYSTEM ADJUSTMENT (A)  MEASUREMENT (Q)  ANALYSIS (A)  HELP (H)

ID:
NAME:

CHANNELS TO BE SELECTED

SELECT ALL CHANNELS

MEASURE    RESET — 100

— 101

SAMPLING
TIME  30 sec ▼
INTERVAL  0.5 msec ▼

MEASURING DIRECTION
○ FRONT SURFACE   ● BACK SURFACE
PRECISE SETTING >>

102

REF. CHANNEL
10 Ch ▼

EP 1 312 304 B1

# FIG. 22

MAGNETIC FILED-STRENGTH MAP

EP 1 312 304 B1

# FIG. 23

### DISPLAY OF TIME-INTEGRATION DIAGRAMS

□ MULTICHANNEL MCG SYSTEM   [?][X]

FILE (F)   LIST OF TO-BE-TESTED PERSONS (L)   DISPLAY (V)   SYSTEM ADJUSTMENT (A)   MEASUREMENT (Q)   ANALYSIS (A)   HELP (H)

ID:
NAME:

100 · 140 msec    180 · 240 msec

CHANNELS TO BE SELECTED
SELECT ALL CHANNELS

COMPONENTS TO BE DISPLAYED
⬤ NORMAL COMPONENT
◯ TANGENTIAL COMPONENT

RE-CONSTITUTED PARAMETER
MAX. VALUE 200 fTsec ◆
INTERVAL 2 fTsec ◆
☑ DISPLAY OF DIFFERENCE —147

UPDATE   CANCEL —134
135

REF. CHANNEL
10 Ch ▼

SCALE
TIME 100 msec ▼
FLUX DENSITY 20 pT ▼

145-2  145-1   146-1  146-2

EP 1 312 304 B1

# FIG. 24

DISPLAY OF SUMMARY

# FIG. 25

DIAGRAM OF EFFECT

## FIG. 26

EFFECTIVE PARAMETERS FOR EACH OF THE PATIENTS

| | NUMBER OF CASES | INTERVAL BETWEEN PEAKS OF P-WAVE AND R-WAVE | CURRENT DIRECTION OF R-WAVE | CURRENT DIRECTION OF T-WAVE | CURRENT DIRECTION OF R-WAVE | MAX. VALUE OF ST-T TIME INTEGRATION | MIN. VALUE OF ST-T TO QRS INTEGRATION | SUM OF QRS VALUES |
|---|---|---|---|---|---|---|---|---|
| STRICTURE OF THE HEART | 7 | | | ◎ | | | | |
| MYOCARDIAL INFARCTION | 11 | | | ◎ | | | | |
| WPW SYNDROME | 6 | ◎ | | | | | | |
| LEG BLOCK | 4 | ◎ | | | ◎ | | | |
| CONTRACTION OUTSIDE OF VENTRICLE | 2 | ◎ | | ◎ | | ◎ | ◎ | ◎ |
| CORONARY VEINS-SINUS RHYTHM | 4 | | ◎ | | | | | |

EP 1 312 304 B1

# FIG. 27

DATA FLOW DIAGRAM (BLOCK DIAGRAM)

EP 1 312 304 B1

# FIG. 28

NEURON

# FIG. 29

NEURAL NETWORK OF
HIERARCHICAL STRUCTURE

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3140731 B
- JP 3140732 B
- US 6195576 B1
- WO 9955228 A

### Non-patent literature cited in the description

- **YOSHIKO HASEGAWA.** Improving the Reliability of the Overall Judgement in the Health Examination by Using MTS. *Quality Engineering,* April 1999, vol. 7 (2